(19) Europäisches Patentamt
European Patent Office
Office européen des brevets

(11) EP 4 199 970 B1

(12) **EUROPEAN PATENT SPECIFICATION**

(45) Date of publication and mention of the grant of the patent:
**15.10.2025 Bulletin 2025/42**

(21) Application number: **21765885.5**

(22) Date of filing: **18.08.2021**

(51) International Patent Classification (IPC):
***A61K 47/68** (2017.01)* ***A61K 51/04** (2006.01)*
***A61K 51/06** (2006.01)* ***A61M 1/36** (2006.01)*

(52) Cooperative Patent Classification (CPC):
**A61K 47/6897; A61K 51/0495; A61K 51/065; A61M 1/3615; A61M 1/3687**

(86) International application number:
**PCT/EP2021/072932**

(87) International publication number:
**WO 2022/038183 (24.02.2022 Gazette 2022/08)**

(54) **EXTRACORPOREAL CLEARING TRAPS BASED ON INVERSE ELECTRON DEMAND DIELS-ALDER CYCLOADDITION FOR (PRE)-TARGETED THERAPY AND DIAGNOSTICS**

EXTRAKORPORALE CLEARING-FALLEN BASIEREND AUF DER INVERSEN ELEKTRONENBEDARF-DIELS-ALDER-CYCLOADDITION FÜR (PRÄ-)TARGETED THERAPIE UND DIAGNOSTIK

PIÈGES DE DÉGAGEMENT EXTRACORPOREL BASÉS SUR LA CYCLOADDITION DIELS-ALDER À DEMANDE INVERSE D'ÉLECTRON POUR UNE THÉRAPIE ET UN DIAGNOSTIC (PRÉ) CIBLÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.08.2020 EP 20191448**

(43) Date of publication of application:
**28.06.2023 Bulletin 2023/26**

(73) Proprietors:
- **Rigshospitalet**
  **2100 Copenhagen Ø (DK)**
- **University of Copenhagen**
  **2100 Copenhagen Ø (DK)**

(72) Inventors:
- **KJÆR, Andreas**
  **2000 Frederiksberg (DK)**
- **HERTH, Matthias Manfred**
  **21121 Malmö (SE)**
- **SHALGUNOV, Vladimir**
  **2820 Gentofte (DK)**
- **JØRGENSEN, Jesper Tranekjær**
  **2100 Copenhagen Ø (DK)**

(74) Representative: **Budde Schou A/S**
**Dronningens Tvaergade 30**
**1302 Copenhagen K (DK)**

(56) References cited:
**EP-A1- 1 346 730** **US-A1- 2019 247 513**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 199 970 B1

## Description

FIELD OF THE INVENTION

The present invention relates to extracorporeal removal of targeting vectors applied in pretargeted therapy and diagnostics in animals and humans. The means for extracorporeal removal of the targeting vectors is based on binding agents with inverse electron demand Diels-Alder (IEDDA) cycloaddition reactivity. The targeting vector comprises a therapeutic agent, a diagnostic agent or a theranostic agent and a chemical entity with IEDDA reactivity whereas the extracorporeal means comprises a column with a biocompatible solid support to which a chemical entity with complementary IEDDA reactivity is attached directly or via a linker. Extracorporeal removal of targeting vectors increases the efficiency of the therapy and/or diagnosis by removal of excess circulating targeting vectors whereby the targeting vectors that are bound to the target becomes easier to identify due to less tumor-to-background ratio. Moreover, the off-site toxicity of the targeting vector is reduced.

BACKGROUND OF THE INVENTION

In many areas of medical diagnosis and therapy, it is desired to selectively deliver an agent, such as a therapeutic agent or a diagnostic agent such as an imaging agent, to a specific site, or a confined region, in the body of a subject such as a human patient or a mammal.

Targeting of an organ or a tissue is achieved by the direct or indirect conjugation of the desired active moieties such as contrast-enhancing agents and cytotoxic compounds to a targeting vector, which binds to cell surfaces or promotes cellular uptake at or near the target site of interest. The targeting moieties used to target such agents are typically constructs that have affinity for cell surface targets such as membrane receptors, structural proteins such as amyloid plaques, or intracellular targets such as RNA, DNA, enzymes and cell signaling pathways.

Targeting can also be based on the propensity of the targeting vector to passively accumulate at or near the target site due to alterations in the structure in the target site (e.g. due to the enhanced permeability and retention effect in solid tumors).

Targeting vectors can be antibodies (fragments), proteins, aptamers, synthetic polymers, oligopeptides, oligonucleotides, oligosaccharides, as well as peptides, peptoids and synthetic drug compounds.

An important criterion for successful molecular imaging and/or therapy agents in general and nuclear imaging and/or therapy agents in particular is that they exhibit a high target uptake while showing a rapid clearance through renal and/or hepatobiliary systems from non-target tissue and from the blood. However, this is criterion is often difficult or impossible to meet. Imaging studies in humans have for example shown that whereas the maximum concentration of a radiolabeled antibody at the tumor site is attainable within 24 h several more days are required before the concentration of the labeled antibody in circulation decreases to levels low enough for successful imaging to take place.

Prolonged circulation through non-target tissues of the therapeutic agent such as a radionuclide or drug conjugated to the targeting vector leads to off-site toxicity and decreases the therapeutic index of the agent.

The problem of slow or insufficient accumulation of the therapeutic agent in target tissue and slow clearance from non-target areas can be mitigated by the application of pretargeting approaches.

In pretargeting approaches (as illustrated in Figure 1), the targeting vector is modified with a tag which enables fast and specific binding to a small-molecular therapeutic or diagnostic agent (subsequently identified as the effector probe). In the first step, unlabeled tagged targeting vector is administered to a subject and given time to accumulate at the disease site usually for 3-7 days. In the second step, the effector probe is administered to the subject. The effector probe binds to the targeting vector upon encountering it, and thus accumulates at the target site. Unbound effector probe is excreted from the body, minimizing the exposure of healthy tissues to effector probe's action which can be radiation, cytotoxicity, reactive oxygen species generation etc.

Mechanisms of selective vector-probe interaction applied in pretargeting include antibody-hapten, or biotin-(strept)avidin binding, or covalent click ligation based on the inverse electron demand Diels-Alder (IEDDA) cycloaddition of dienes and dienophiles, among others. These first two mechanisms have been evaluated in clinical trials, but have not entered routine clinical practice, due to immunogenicity issues towards avidin and lack of modularity with bispecific antibodies.

IEDDA cycloaddition is the most promising pretargeting strategy, which is based on a chemical reaction with -extremely fast kinetics (bimolecular reaction rate up to ~ $10^8$ $M^{-1}s^{-1}$). Furthermore, the reaction is selective in vivo ("bioorthogonal"), while relevant reactive moieties can be easily attached to a wide range of substrates.

A general description of IEDDA-reactive dienes (e.g. tetrazines) and dienophiles (e.g. trans-cyclooctenes) are disclosed in US 9 913 921 B2. The substitution pattern of tetrazines is known to influence the kinetics of their reaction with trans-cyclooctenes. In particular, tetrazines substituted with electron-donating groups, such as methyl-substituted tetrazines, react with trans-cyclooctenes at >10-fold slower rate than unsubstituted tetrazines or tetrazines substituted

with electron-withdrawing groups, such as (bis)pyridyl (Oliveira, B. L.; Guo, Z.; Bernardes, G. J. L. Inverse Electron Demand Diels-Alder Reactions in Chemical Biology. Chem. Soc. Rev. 2017, 46 (16), 4895-4950; Stéen, E. J. L.; Jørgensen, J. T.; Denk, C.; Battisti, U. M.; Nørregaard, K.; Edem, P. E.; Bratteby, K.; Shalgunov, V.; Wilkovitsch, M.; Svatunek, D.; et al. Lipophilicity and Click Reactivity Determine the Performance of Bioorthogonal Tetrazine Tools in Pretargeted In Vivo Chemistry. ACS Pharmacol. Transl. Sci. 2021, 4 (2), 824-833.).

Even with pretargeting approaches, the presence of circulating targeting vector in the blood is a factor that limits the delivery of the effector probe to the target disease site and contributes to off-target toxicity (as shown in Figure 2A). This is the case for all state-of-the-art pretargeting strategies.

Approaches exist to remove circulating targeting vector from the bloodstream. One approach is to use "clearing agents" which are injectable compounds whose biodistribution is restricted to the blood pool. Clearing agents are capable of binding to the targeting vectors in the same fashion as effector probes do, and contain moieties recognized by the liver or by the immune system. By attaching themselves to the circulating targeting vectors, clearing agents accelerate the removal of the latter from the bloodstream. Such agents have been applied to monoclonal antibody (mAb) based targeting vectors with variable success. However, clinical translation of clearing agents is challenging due to high doses necessary to achieve efficient clearing and unclear properties of the resulting targeting vector conjugate.

Another approach is extracorporeal clearing as illustrated in Figure 2B. In this approach, the blood of the subject whereto the targeting vector has been administered is passed through a solid resin support, decorated with moieties that recognize and bind the targeting vector molecules in the same fashion as effector probes do. After passing through the solid support, the blood, from which the present targeting vector has been removed, is returned into the patient. Extracorporeal clearing based on biotin-avidin interaction has been employed to improve tumor-vs-healthy tissue contrast (sometimes referred to as tumor-to-background ratio (TBR)) in conventional and pretargeted radio immunotherapy. Toxicological concerns related to extracorporeal clearing are significantly reduced compared to the use of injectable clearing agents since no clearing compounds have to be injected into the body.

Extracorporeal clearing approach developed for a certain pretargeting pair (biotin-streptavidin, antibody-hapten etc.) is not directly translatable to a different pair, because the interactions in each pair are highly selective. To date, no extracorporeal clearing approach based on IEDDA chemistry has been demonstrated.

Extracorporeal clearing based on IEDDA chemistry will possess the same advantages over other interactions used for extracorporeal clearing as are intrinsic to IEDDA reaction, namely: more efficient trapping due to fast kinetics and high resin loadings possible for small IEDDA-reactive compounds, lack of cross-reactivity with endogenous molecules and easy modification of vectors that need to be cleared.

Document US2019247513 is part of the prior art.

## SUMMARY OF THE INVENTION

The present invention provides an extracorporeal clearing trap column comprising a biocompatible solid support to which a chemical entity is attached, characterized in that the chemical entity possesses inverse electron demand Diels-Alder cycloaddition reactivity. The extracorporeal clearing trap is suitable for removing from a bloodstream targeting vectors comprising a therapeutic or diagnostic agent and a chemical entity with IEDDA reactivity complementary to the chemical entity possessing IEDDA reactivity in the clearing trap.

The present invention also provides a chemical entity with inverse electron demand Diels-Alder cycloaddition reactivity attached to an extracorporeal clearing trap column for use in the extracorporeal treatment of a disease wherein a targeting vector, comprising a therapeutic or diagnostic agent or a part of such agent and a complementary to the chemical entity in the clearing trap, has been administered to a subject.

The present disclosure not part of the invention further provides a method for implementation of the extracorporeal clearing approach based on inverse electron demand Diels-Alder (IEDDA) cycloaddition chemistry and a clearing trap column comprising a biocompatible solid support to which chemical moieties capable of IEDDA cycloaddition complementary to the IEDDA reactivity of the targeting vector to be trapped are attached. These chemical moieties possessing the IEDDA reactivity applied in the column are dienes or dienophiles.

The method according to the disclosure not being part of the invention removes circulating targeting vectors comprising a therapeutic agent, a diagnostic agent, a synthetic agent, or part of such an agent and being tagged with IEDDA-reactive moieties from the bloodstream of a human patient or other mammals by passing the blood through a solid support bearing complementary IEDDA-reactive moieties and afterward, returning the blood back into the subject.

The present invention moreover relates to use of a clearing trap for removing from a bloodstream targeting vectors comprising a therapeutic or diagnostic agent and a chemical entity with IEDDA reactivity complementary to the chemical entity possessing IEDDA reactivity in the clearing trap. The method according to the disclosure not being part of the invention increases the efficiency of pretargeting approaches based on IEDDA cycloaddition by ensuring selective delivery of the effector probe to the target site through the removal of circulating targeting vectors from the bloodstream by means of the clearing trap column according to the invention.

BRIEF DESCRIPTION OF THE DRAWINGS

Figure 1: The pretargeting principle.

Figure 2: The extracorporeal clearing principle. The body of the subject is represented by the disease site (tumor), the heart, and blood circulating between them. 2A shows the pretargeting approach without clearing; 2B shows the pretargeting approach when extracorporeal clearing is applied.

Figure 3: Chemical structures of six amino-tetrazine derivatives tested as trapping agents.

Figure 4: Amino-tetrazine derivative 7 and its $^{111}$In-labeled form [$^{111}$In]8.

Figure 5: Example of HPLC radio-chromatogram of [$^{111}$In]8.

Figure 6: Structure of a TCO-decorated monoclonal antibody and a TCO-decorated polymer used for determining the trapping efficiency of the agarose resin modified with the amino-tetrazine derivatives.

Figure 7: Examples of HPLC chromatograms of amino-tetrazines 1-6 (Figures 7A to 7F, respectively).

Figure 8: Examples of SDS-PAGE gel autoradiograms obtained from the titration of TCO-CC49 and TCO-KS254 with [$^{111}$In]8.

Figure 9: Diagrams of trapping efficiencies for the agarose resin modified with the amino-tetrazine derivatives. A - trapping efficiencies for TCO-CC49, B - trapping efficiencies for TCO-KS254. Bars show mean values from 2 independent experiments, error bars show standard deviations.

Figure 10: Scheme of the extracorporeal trapping circuit used for in vivo experiments.

Figure 11: Example of HPLC radio-chromatogram of [$^{111}$In]TCO-CC49.

Figure 12: Radioactivity uptake in the blood and organs of rats injected with [$^{111}$In]TCO-CC49 and subjected to extracorporeal clearing procedure using tetrazine-decorated and tetrazine-free (sham) agarose columns.

DETAILED DESCRIPTION OF THE INVENTION

In a first aspect, the present disclosure not being part of the invention provides a method for extracorporeal removal of a targeting vector, comprising a therapeutic or diagnostic agent or a part of such agent, and to which the first chemical entity with IEDDA reactivity is attached; comprising:

a) Administering the targeting vector to an animal,
b) Passing the bloodstream of the animal through a clearing trap column comprising a biocompatible solid support to which a second chemical entity possessing IEDDA reactivity complementary to the first chemical entity of the targeting vector is attached
c) Returning the bloodstream to the animal.

The method, not being part of the invention, is suitable for any animal but is particularly relevant in the treatment of mammals, preferably humans. The term "patient" refers in the context of the present disclosure wherein the method is not part of the invention, to an animal, particular to a human, subject to treatment, diagnostics or theranostics wherein a pretargeting approach is applied. Theranostic agents are agents suitable for use both as a diagnostic agent and as a therapeutic agent.

The general principle of extracorporeal clearing is shown in Figure 2B. In context of the method provided in the first aspect of this disclosure; the method not being part of the invention; a targeting vector comprising a therapeutic or diagnostic agent or a part of such agent, and a first chemical entity with IEDDA reactivity is administered to a patient. The targeting vector must be allowed sufficient time for the targeting vector to reach the target. The sufficient time period will depend on the vector and on the target and will be estimated in early clinical trials of the specific vector. After allowing sufficient time for the vector to reach the target, the blood stream of the patient is passed through the clearing trap column.

The clearing trap column comprises a biocompatible support to which a second chemical entity with IEDDA reactivity complementary to the first chemical entity of the targeting vector is attached. When the bloodstream is passed through the column, the second chemical entity of the clearing trap will recognize and bind to the first chemical entity of the targeting vector thereby trapping the targeting vector in the clearing trap column. After passing through the solid support, the blood, from which the targeting vector has been removed, is returned into the patient. Subsequent administration of the effector probe will thus be improved because binding of the effector probe will be restricted to the target vectors that have reached the target.

Any therapeutic agent suitable for pretargeting therapeutic approaches could be used in the present method. As this approach is developing rapidly, it is expected that more and more therapeutic agents will be tested and found to be suitable for pretargeting therapeutic approaches. Presently, the approach is mainly applied within cancer diagnosis, surgery and therapy.

The extracorporeal clearing aspect is based in binding agents possessing IEDDA reactivity. In order for the method to work, the IEDDA reactivity of the targeting vector and of the clearing trap must be complementary. Pairs of chemical entities with complementary IEDDA reactivity such as dienes and dienophiles are well known in the art and includes *trans*-cyclooctenes (TCO) and s-tetrazines.

In one embodiment, the chemical entity of the targeting vector is a dienophile and the chemical entity of the column is a diene.

In another embodiment, the chemical entity of the targeting vector is a diene and the chemical entity of the clearing trap column is a dienophile.

In preferred embodiments, the diene is a 1,2,4,5-tetrazine derivative and the dienophile is a *trans*-cyclooctene derivative.

Particularly preferred embodiments are embodiments wherein the diene derivative as either the first chemical entity of the target vector or as the second chemical entity of the clearing trap column is selected from the group comprising:

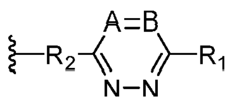

wherein:

A and B are independently N or CH

$R_1$ is H or a $C_1$-$C_{10}$ alkyl or is selected from the group consisting of:

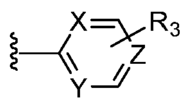

wherein X, Y and Z are independently N or CH;

$R_3$ is a $C_1$-$C_{10}$ alkyl, fluorine, bromine, iodine, carboxylic acid, -CO-$NH_2$, -NH-CO-H, -NH-CO-$CH_3$, or -$OR_4$, wherein $R_4$ is H, or a $C_1$-$C_{10}$ alkyl or -[-$CH_2$-$CH_2$-O-]$_n$-$CH_3$ wherein n is an integer selected from the group consisting of 1, 2, 3, 4, 5, 6, 7, 8, 9 and 10;

$R_2$ is a $C_1$-$C_{10}$ alkyl, -[-$CH_2$-$CH_2$-O-]$_m$-, wherein in m is an integer selected from the group consisting of 1, 2, 3, 4, 5, 6, 7, 8, 9 and 10, or is selected from the group consisting of:

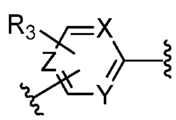

wherein X, Y and Z are independently N or CH;

$R_3$ is a $C_1$-$C_{10}$ alkyl, fluorine, bromine, iodine, carboxylic acid, -CO-$NH_2$, -NH-CO-H, -NH-CO-$CH_3$, or -$OR_4$, wherein $R_4$ is H, or a $C_1$-$C_{10}$ alkyl or -[-$CH_2$-$CH_2$-O-]$_n$-$CH_3$, wherein n is an integer selected from the group consisting of 1, 2, 3, 4, 5, 6, 7, 8, 9 and 10;

and wherein the complementary trans-cyclooctene derivative is selected from the group comprising:

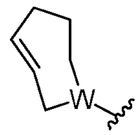

wherein W is $-CH_2-CH(-O-)-$, $-CH_2-CH(-NH-)-$, -C(=O)-N(-)-, -N(-)-C(=O)-, a fused 3-membered ring selected from the group consisting of:

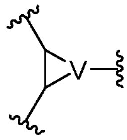

wherein V is N or CH,

a fused 4-membered ring selected from the group consisting of:

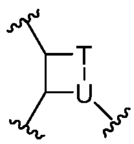

wherein T is O, NH, $CH_2$ or C=O and

U is -CH- or -N-,

or a fused 5-membered ring selected from the group consisting of:

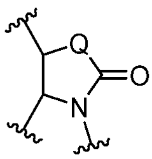 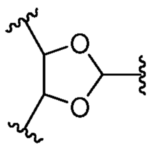 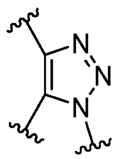

Wherein Q is O, S or NH.

In another preferred embodiment, the diene derivative as either the first chemical entity of the target vector or as the second chemical entity of the clearing trap column is an 1,2,4,5-tetrazine derivative selected from the group comprising:

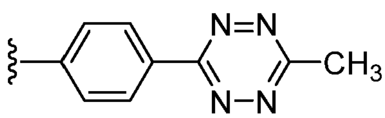

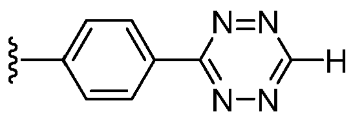

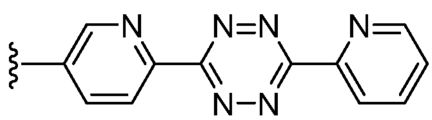

and wherein the dienophile is a trans-cyclooctene derivative selected from the group comprising:

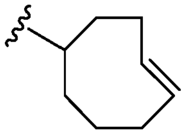 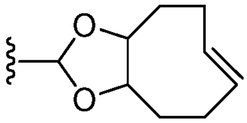 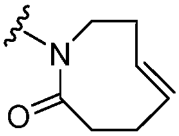 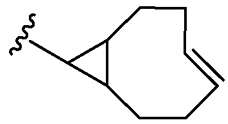.

The therapeutic, diagnostic or theranostic probe may be any suitable probe providing a therapeutic effect, a visual effect or both.

In one embodiment, the therapeutic, diagnostic agent or theranostic agent is an optical probe such as fluorescein, indocyanine green, fluorescein isothiocyanate, carboxyfluorescein, rhodamine derivatives, coumarin derivatives cyanine derivatives, Cy5.5, Alexa 680, Cy5, DiD (1,1'-dioctadecyl-3,3,3',-3-tetramethylindodicarbocyanine perchlorate), and DiR (1,1'-dioctadecyl-3,3,3',-3'-tetramethylindotricarbocyanine iodide).

In another embodiment, the therapeutic, diagnostic agent or theranostic agent is a fluorescent protein such as green fluorescent protein (GFP), Yellow fluorescent protein (YFP), Red fluorescent protein (RFP).

In yet another embodiment, therapeutic, diagnostic agent or theranostic agent is a radioisotope such as $^{3}H$, $^{11}C$, $^{13}N$, $^{18}F$, $^{19}F$, $^{60}Co$, $^{64}Cu$, $^{68}Ga$, $^{82}Rb$, $^{90}Sr$, $^{90}Y$, $^{99}Tc$, $^{99m}Tc$, $^{111}In$, $^{123}I$, $^{124}I$, $^{125}I$, $^{129}I$, $^{131}I$, $^{137}Cs$, $^{177}Lu$, $^{186}Re$, $^{188}Re$, $^{211}At$, $^{225}Ac$, Rn, Ra, Th, U, Pu and $^{241}Am$.

In yet another embodiment, therapeutic, diagnostic agent or theranostic agent is an antibody such as vancomycin, paclitaxel, doxorubicin, etoposide, irinotecan, SN-38, cyclosporin A, podophyllotoxin, carmustine, amphotericin, ixabepilone, patupilone, rapamycin; a platinum drug or other drugs such as doxycylin, MMP inhibitors, daptomycin L-dopa, oseltamivir, cephalexin, 5-aminolevulinic acid, cysteine, nystatin, amphotericin B, flucytosine, emtricibatine, trimethoprim and sulfamecetriazone.

The targeting vector comprising the therapeutic, diagnostic agent or theranostic agent and a chemical entity possessing IEDDA reactivity can be any vector known in the art suitable for pretargeting approaches. Such targeting vectors includes small molecules, antibodies, aptamers, nanoparticles and polymers.

In preferred embodiments, the targeting vector is a monoclonal antibody or a polypeptoid polymer.

In a second aspect, the present invention provides an extracorporeal clearing trap column that is applicable for use in the method provided in the first aspect.

In a third aspect, the present invention provides a chemical entity with inverse electron demand Diels-Alder cycloaddition reactivity attached to an extracorporeal clearing trap column for use in the extracorporeal treatment of a disease wherein a targeting vector, comprising a therapeutic or diagnostic agent or a part of such agent and a complementary to the chemical entity in the clearing trap, has been administered to a subject.

The chemical entity possessing IEDDA reactivity is attached to a biocompatible solid support of the extracorporeal clearing trap column.

The extracorporeal clearing trap column thus comprises a biocompatible solid support to which a chemical entity possessing IEDDA reactivity is attached. The chemical entity possessing IEDDA reactivity may be attached directly to the biocompatible solid support or by a linker.

In one embodiment, a clearing trap column is provided that comprises a biocompatible solid support to which a chemical entity possessing IEDDA reactivity is attached, for use in the extracorporeal treatment of a disease wherein a targeting vector comprising a therapeutic or diagnostic agent and a chemical entity with IEDDA reactivity complementary to the chemical entity in the clearing trap, has been administered to a patient.

In another embodiment, an extracorporeal clearing trap column is provided that comprises a biocompatible solid support to which a chemical entity possessing IEDDA reactivity is attached, for use in the extracorporeal treatment of a disease wherein a targeting vector comprising a therapeutic or diagnostic agent or a part of such agent and a chemical entity with IEDDA reactivity complementary to the chemical entity in the clearing trap, has been administered to a patient.

The chemical entity with IEDDA reactivity must be complementary to the IEDDA reactivity of the targeting vector. Complementary pairs of chemical entities with IEDDA reactivity is well known in the art, and the skilled person will be able to select complementary pairs.

In one embodiment, the chemical entity of the targeting vector is a diene and the chemical entity in the clearing trap column is a dienophile.

In another embodiment, the chemical entity of the targeting vector is a diene and the chemical entity in the column is a dienophile.

In preferred embodiments, the diene is an 1,2,4,5-tetrazine derivative and the dienophile is a trans-cyclooctene derivative.

Particularly preferred embodiments are embodiments wherein the diene as either the first chemical entity of the target vector or the second chemical entity of the clearing trap column is selected from the group comprising:

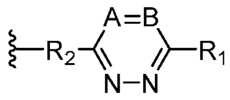

wherein:

A and B are independently N or CH

$R_1$ H or $C_1$-$C_{10}$ alkyl or is selected from the group consisting of:

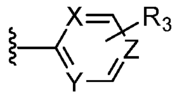

wherein X, Y, Z are independently N or CH,

$R_3$ is $C_1$-$C_{10}$ alkyl, fluorine, bromine, iodine, carboxylic acid, -CO-$NH_2$, -NH-CO-H, -NH-CO-$CH_3$, -O-$R_4$, wherein $R_4$ is H, or $C_1$-$C_{10}$ alkyl or -[-$CH_2$-$CH_2$-O-]$_n$-$CH_3$ wherein n is an integer selected from the group consisting of 1, 2, 3, 4, 5, 6, 7, 8, 9 and 10;

$R_2$ is $C_1$-$C_{10}$ alkyl, -[-$CH_2$-$CH_2$-O-]$_m$-, wherein in m is an integer selected from the group consisting of 1, 2, 3, 4, 5, 6, 7, 8, 9 and 10, or is selected from the group consisting of:

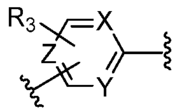

wherein X, Y, Z are independently N or CH,

$R_3$ is $C_1$-$C_{10}$ alkyl, fluorine, bromine, iodine, carboxylic acid, -CO-$NH_2$, -NH-CO-H, -NH-CO-$CH_3$, -O-$R_4$, wherein $R_4$ is H, or $C_1$-$C_{10}$ alkyl or -[-$CH_2$-$CH_2$-O-]$_n$-$CH_3$ wherein n is an integer selected from the group consisting of 1, 2, 3, 4, 5, 6, 7, 8, 9 and 10;

and wherein the dienophile complementary to the diene as either the first chemical entity of the target vector or the second chemical entity of the clearing trap is a trans-cyclooctene derivative selected from the group comprising:

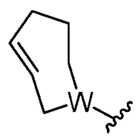

wherein W is -$CH_2$-CH(-O-)-, or -$CH_2$-CH(-NH-)-, or -C(=O)-N(-)-, -N(-)-C(=O)-, a fused 3-membered ring selected from the group consisting of:

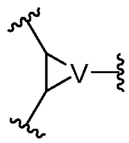

wherein V is N or CH,

a fused 4-membered ring selected from the group consisting of:

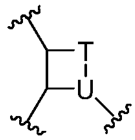

wherein T is O, NH, $CH_2$ or C=O and

U is -CH- or -N-,

or a fused 5-membered ring selected from the group consisting of:

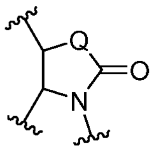 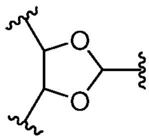 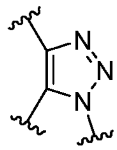

Wherein Q is O, S or NH

In another preferred embodiment, the diene as either the first chemical entity of the target vector or the second chemical entity of the clearing trap column is a 1,2,4,5-tetrazine derivative selected from the group comprising:

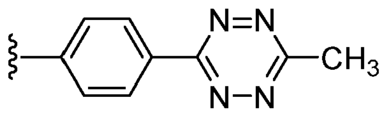

,

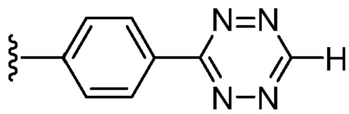

and

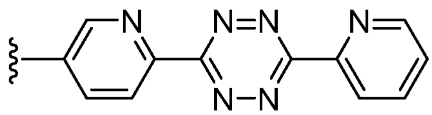

and wherein the dienophile complementary to the diene as either the first chemical entity of the target vector or the second chemical entity of the clearing trap is a trans-cyclooctene derivative selected from the group comprising:

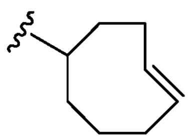

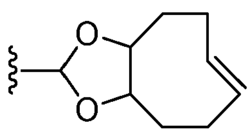

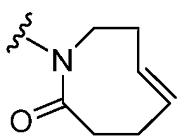

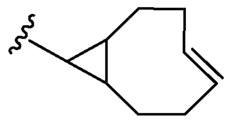

In embodiments wherein the chemical entity with inverse electron demand Diels-Alder cycloaddition reactivity attached to an extracorporeal clearing trap column for use in the extracorporeal treatment of a disease wherein a targeting vector, comprising a therapeutic or diagnostic agent or a part of such agent and a complementary to the chemical entity in the clearing trap, has been administered to a subject, the therapeutic, diagnostic or theranostic probe may be any suitable probe providing a therapeutic effect, a visual effect or both.

In one embodiment, the therapeutic, diagnostic agent or theranostic agent is an optical probe such as fluorescein, indocyanine green, fluorescein isothiocyanate, carboxyfluorescein, rhodamine derivatives, coumarin derivatives cyanine derivatives, Cy5.5, Alexa 680, Cy5, DiD (1,1'-dioctadecyl-3,3,3',-3-tetramethylindodicarbocyanine perchlorate), and DiR (1,1'-dioctadecyl-3,3,3',-3'-tetramethylindotricarbocyanine iodide).

In another embodiment, the therapeutic, diagnostic agent or theranostic agent is a fluorescent protein such as green fluorescent protein (GFP), Yellow fluorescent protein (YFP), Red fluorescent protein (RFP).

In yet another embodiment, therapeutic, diagnostic agent or theranostic agent is a radioisotope such as $^{3}H$, $^{11}C$, $^{13}N$, $^{18}F$, $^{19}F$, $^{60}Co$, $^{64}Cu$, $^{68}Ga$, $^{82}Rb$, $^{90}Sr$, $^{90}Y$, $^{99}Tc$, $^{99m}Tc$, $^{111}In$, $^{123}I$, $^{124}I$, $^{125}I$, $^{129}I$, $^{131}I$, $^{137}Cs$, $^{177}Lu$, $^{186}Re$, $^{188}Re$, $^{211}At$, $^{225}Ac$, Rn, Ra, Th, U, Pu and $^{241}Am$.

In yet another embodiment, therapeutic, diagnostic agent or theranostic agent is an antibody such as vancomycin, paclitaxel, doxorubicin, etoposide, irinotecan, SN-38, cyclosporin A, podophyllotoxin, carmustine, amphotericin, ixabepilone, patupilone, rapamycin; a platinum drug or other drugs such as doxycylin, MMP inhibitors, daptomycin L-dopa, oseltamivir, cephalexin, 5-aminolevulinic acid, cysteine, nystatin, amphotericin B, flucytosine, emtricibatine, trimethoprim and sulfamecetriazone.

The targeting vector comprising the therapeutic, diagnostic agent or theranostic agent and a chemical entity possessing IEDDA reactivity can be any vector known in the art suitable for pretargeting approaches. Such targeting vectors includes small molecules, antibodies, aptamers, nanoparticles and polymers.

In preferred embodiments, the targeting vector is a monoclonal antibody or a polypeptoid polymer.

In embodiments wherein the extracorporeal clearing trap column comprising a biocompatible solid support to which a chemical entity possessing inverse electron demand Diels-Alder cycloaddition reactivity is attached is used in the extracorporeal treatment of a disease wherein a targeting vector, comprising a therapeutic or diagnostic agent or a part of such agent and a complementary to the chemical entity in the clearing trap, has been administered to a subject, the therapeutic, diagnostic or theranostic probe may be any suitable probe providing a therapeutic effect, a visual effect or both.

In one embodiment, the therapeutic, diagnostic agent or theranostic agent is an optical probe such as fluorescein, indocyanine green, fluorescein isothiocyanate, carboxyfluorescein, rhodamine derivatives, coumarin derivatives cyanine derivatives, Cy5.5, Alexa 680, Cy5, DiD (1,1'-dioctadecyl-3,3,3',-3-tetramethylindodicarbocyanine perchlorate), and DiR (1,1'-dioctadecyl-3,3,3',-3'-tetramethylindotricarbocyanine iodide).

In another embodiment, the therapeutic, diagnostic agent or theranostic agent is a fluorescent protein such as green fluorescent protein (GFP), Yellow fluorescent protein (YFP), Red fluorescent protein (RFP).

In yet another embodiment, therapeutic, diagnostic agent or theranostic agent is a radioisotope such as $^{3}H$, $^{11}C$, $^{13}N$, $^{18}F$, $^{19}F$, $^{60}Co$, $^{64}Cu$, $^{68}Ga$, $^{82}Rb$, $^{90}Sr$, $^{90}Y$, $^{99}Tc$, $^{99m}Tc$, $^{111}In$, $^{123}I$, $^{124}I$, $^{125}I$, $^{129}I$, $^{131}I$, $^{137}Cs$, $^{177}Lu$, $^{186}Re$, $^{188}Re$, $^{211}At$, $^{225}Ac$, Rn, Ra, Th, U, Pu and $^{241}Am$.

In yet another embodiment, therapeutic, diagnostic agent or theranostic agent is an antibody such as vancomycin, paclitaxel, doxorubicin, etoposide, irinotecan, SN-38, cyclosporin A, podophyllotoxin, carmustine, amphotericin, ixabepilone, patupilone, rapamycin; a platinum drug or other drugs such as doxycylin, MMP inhibitors, daptomycin L-dopa, oseltamivir, cephalexin, 5-aminolevulinic acid, cysteine, nystatin, amphotericin B, flucytosine, emtricibatine, trimethoprim and sulfamecetriazone.

The targeting vector comprising the therapeutic, diagnostic agent or theranostic agent and a chemical entity possessing IEDDA reactivity can be any vector known in the art suitable for pretargeting approaches. Such targeting vectors includes small molecules, antibodies, aptamers, nanoparticles and polymers.

In preferred embodiments, the targeting vector is a monoclonal antibody or a polypeptoid polymer.

The biocompatible solid support of the clearing trap column can be any suitable biocompatible solid support can be used in the method not being part of the present disclosure.

For example, the biocompatible solid support can be a hydrogel, a cross linked polymer matrix, a metal, a ceramic, or a plastic.

Suitable hydrogels in the present invention include, but are not limited to, polysaccharide hydrogels, agarose,

alginate, cellulose, hyaluronic acid, chitosan, chitosin, chitin, hyaluronic acid, chondroitin sulfate, and heparin.

Other Sugar-based biomaterials that are suitable as biocompatible solid support in the clearing trap column of the present invention are known in the art. Examples are those described in Polymer Advanced Technology 2014, 25, 448-460.

Suitable polymers as the biocompatible solid support in the clearing trap column of the present invention include, but are not limited to, polyphosphazenes, polyanhydrides, polyacetals, poly(ortho esters), polyphosphoesters, polycaprolactones, polyurethanes, polylactides, polycarbonates, polyamides, and polyethers, and blends/composites/copolymers thereof. Representative polyethers include, but are not limited to, Poly(ethylene glycol) (PEG), poly(propylene glycol) (PPG), triblock Pluronic (PEGn PPGlm-PEGn), PEG diacrylate (PEGDA) and PEG dimethacrylate (PEGDMA).

The biocompatible solid support in the clearing trap column of the present invention can also include proteins and other poly(amino acids) such as collagen, gelatin, elastin and elastin-like polypeptides, albumin, fibrin, poly(gamma-glutamic acid), poly(L-lysine), poly(L-glutamic acid), and poly(aspartic acid).

In a preferred embodiment, the biocompatible solid support in the clearing trap column of the present invention support is agarose.

The chemical entity with IEDDA reactivity attached to the solid support in the clearing trap column may be attached directly to the solid support or via a linker.

In one embodiment, the chemical entity with IEDDA reactivity attached to the solid support in the clearing trap column is attached to the solid support via a linker.

Any suitable linker can be used in the present invention to link the binding agent to the biocompatible solid support or to the therapeutic or diagnostic agent.

One group of suitable linkers have about 1 to about 100 linking atoms such as from about 1 to about 50 linking atoms, such as from about 1 to about 10 linking atoms, or such as from about 5 to about 10 linking atom.

The types of bonds that can be used to link the chemical entity with IEDDA reactivity of the clearing trap column to the biocompatible support of the clearing trap column include amides, amines, esters, carbamates, ureas, thioethers, thiocarbamates, thiocarbonate and thioureas. However, the skilled person will know that other types of linkers may be applicable.

In some embodiments, the linker includes one or more ethylene-oxy moieties, amines, esters, amides, ketone, urea, carbamate and carbonate functional groups.

Preferably, the linker includes one or more of an amide bond, a urea bond, an alkane chain, a polypeptide, a polypeptoid polymer, polyethylene glycol, N-(2-hydroxypropyl)methacrylamide, polysarcosine, a thiosuccinimide ring or a triazole ring as structural elements connecting the chemical entity possessing IEDDA reactivity to the biocompatible solid support.

In preferred embodiments, the linker is no more than 3 atoms long and contains a single amide bond.

In a third aspect, the present invention provides use of a clearing trap column such as the column according to the second aspect of the invention for removing from a bloodstream targeting vectors comprising a therapeutic or diagnostic agent or a part of such agent and a chemical entity with IEDDA reactivity complementary to the chemical entity possessing IEDDA reactivity in the clearing trap.

In preferred embodiments, the use of a clearing trap column such as the column according to the second aspect of the invention is a use wherein the targeting vector being cleared from the bloodstream is a small molecule, an antibody, a nanoparticle or a polymer.

Also in preferred embodiments, the use of a clearing trap such as the column according to the second aspect of the invention is a use wherein whole blood is passed directly through the trap without prior removal of blood cells by means of a plasma filter or similar device.

Also in preferred embodiments, the use of a clearing trap such as the column according to the second aspect of the invention is a use wherein the therapeutic or diagnostic agent used together with the targeting vector being cleared from the bloodstream is an optical probe such as fluorescein, indocyanine green, fluorescein isothiocyanate, carboxyfluorescein, rhodamine derivatives, coumarin derivatives cyanine derivatives, Cy5.5, Alexa 680, Cy5, DiD (1,1'-dioctadecyl-3,3,3',-3-tetramethylindodicarbocyanine perchlorate), and DiR (1,1'-dioctadecyl-3,3,3',-3'-tetramethylindotricarbocyanine iodide); a fluorescent protein such as green fluorescent protein (GFP), Yellow fluorescent protein (YFP), Red fluorescent protein (RFP); a radioisotope such as 3H, 11C, 13N, 18F, 19F, 60Co, 64Cu, 68Ga, 82Rb, 90Sr, 90Y, 99Tc, 99mTc, 111In, $^{123}$I, 124I, $^{125}$I, 129I, 131I, 137Cs, 177Lu, 186Re, 188Re, 211At, 225Ac, Rn, Ra, Th, U, Pu and 241Am; an antibody such as CC49, B72.3, IDEC-159, a platinum drug, or other drugs such as vancomycin, paclitaxel, doxorubicin, etoposide, irinotecan, SN-38, cyclosporin A, podophyllotoxin, carmustine, ixabepilone, patupilone, rapamycin, doxycylin, MMP inhibitors, daptomycin L-dopa, oseltamivir, cephalexin, 5-aminolevulinic acid, cysteine, nystatin, amphotericin B, flucytosine, emtricibatine, trimethoprim and sulfamecetriazone.

SOURCES

Reviews of pretargeting methods

Altai M, Membreno R, Cook B, Tolmachev V, Zeglis BM. Pretargeted Imaging and Therapy. J Nucl Med 2017;58:1553-9. doi:10.2967/jnumed.117.189944.

Stéen EJL, Edem PE, Nørregaard K, Jørgensen JT, Shalgunov V, Kjaer A, et al. Pretargeting in nuclear imaging and radionuclide therapy: Improving efficacy of theranostics and nanomedicines. Biomaterials 2018;179:209-45. doi:10.1016/j.biomaterials.2018.06.021.

EXAMPLES:

**Example 1: Amino-tetrazines**

Six amino-tetrazines with IEDDA activity were tested for their efficiency as trapping agents for TCO-derivatives with complementary IEDDA activity. The six amino-tetrazines were used for incorporation into agarose hydrogel as described in Example 5.

The formulas of the six amino-tetrazines are shown in Figure 3.

Tetrazine No. 1 and 2 (as shown in Figure 3) were purchased from Jena Bioscience and used without further purification and characterization.

General methods used to prepare and characterize tetrazines 3-7:

All reagents and solvents were purchased from ABX, Sigma Aldrich, Fluorochem and VWR and used as received, without further purification, unless stated otherwise. Dry THF and DCM were obtained from a SG Water solvent purification system and dry dimethyl sulfoxide (DMSO), MeCN, pyridine and methanol (MeOH) were purchased from commercial suppliers. Room temperature corresponds to a temperature interval from 18-21 °C. Reactions requiring anhydrous conditions were carried out under inert atmosphere (nitrogen) and using oven-dried glassware (152 °C).

NMR ($^1$H, $^{13}$C) spectra were acquired on a 600 MHz Bruker Avance III HD, a 400 MHz Bruker Avance II or a Bruker AC200. Samples were measured at 300 K, except for the Bruker AC200, in which samples were measured at 293 K. Chemical shift ($\delta$) are expressed in parts per million and referenced to residual solvent peak. The resonance multiplicity is abbreviated as follows or combinations thereof: s (singlet), bs (broad singlet), d (doublet), t (triplet), p (quintet) and m (multiplet). All $^{13}$C NMR spectra were measured with proton decoupling. Thin-layer chromatography (TLC) was run on silica plated aluminum sheets (Silica gel 60 F254) from Merck and the spots were visualized by ultraviolet light at 254 nm, by anisaldehyde and/or by potassium permanganate staining. Flash column chromatography was carried out manually on silica gel 60 (0.040-0.063 mm). Preparative high-performance liquid chromatography (HPLC) was performed on a Thermo Scientific Dionex 3000 UltiMate instrument connected to a Thermo Scientific Dionex 3000 Diode Array Detector using a Gemini-NX 5$\mu$ RP C18 column (250 $\times$ 21.2 mm) with UV detection at 254 and 280 nm. Mobile phase A (MP A): 0.1% trifluoroacetic acid (TFA) in water (v/v). Mobile phase B (MP B): 0.1% TFA, 10% water in ACN (v/v/v). Flow rate: 20 mL/min. Gradient: 0-30 min, 0$\rightarrow$100% MP B; 30-35 min, 100% MP B.

Tetrazine 3 (as shown in Figure 3) was prepared as follows:

tert-Butyl (4-cyanobenzyl)carbamate:

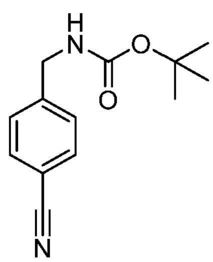

4-(Aminomethyl)benzonitrile hydrochloride (2.00 g, 11.86 mmol, 1 eq.) was dissolved in 200 mL DCM under $N_2$ atmosphere. After the addition of $Boc_2O$ (2.588 g, 11.86 mmol, 1eq.) and $Et_3N$ (5.12ml, 36.76 mmol, 3.1 eq.) the reaction mixture was left to stir at r.t overnight before being concentrated under reduced pressure. The crude was dissolved in EtOAc and washed with sat. $NaHCO_3$ and dried over $MgSO_4$ to afford a white solid (2.22g, 9.55 mmol, 81%). $^1$H NMR (600 MHz, MeOD) $\delta$ 7.68 (d, $J$ = 8.0 Hz, 2H), 7.45 (d, J= 8.0 Hz, 2H), 4.30 (s, 2H), 1.45 (s, 9H). $^{13}$C NMR (151 MHz, MeOD) $\delta$

158.5, 147.0, 133.3, 128.9, 119.7, 111.7, 80.4, 44.6, 28.7.

**tert-Butyl (4-(1,2,4,5-Tetrazin-3-yl)benzyl)carbamate:**

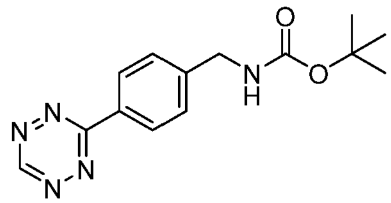

*tert*-Butyl (4-cyanobenzyl)carbamate (3g, 12.91 mmol, 1 eq.), DCM (2.07 mL 32.28 mmol, 2.5 eq.), sulfur (826.27mg, 3.22 mmol, 0.25 eq.) and ethanol (20mL) were mixed together in a 30 mL closed vial. Hydrazine monohydrate (5.86 mL, 103.32 mmol, 8 eq.) was added dropwise while stirring. The vessel was sealed and the reaction mixture was heated to 50 °C for 24 hours. The reaction mixture was cooled to $0^0$C, to which DCM (30 mL), sodium nitrite (8.91g, 129.1 mmol, 10 eq.) and $H_2O$ (100 mL) were added. Excess acetic acid (44.3 mL, 775 mmol, 60 eq.) was added afterwards slowly during which the solution turned bright red in color (pH=3). The reaction mixture was extracted with DCM (150 mL). The organic phase was dried over $MgSO_4$, filtered and concentrated under reduced pressure. The resulting residue was purified using silica gel chromatography (15:85 EtOAc:Heptane) and then fractions containing the desired compound were combined and concentrated (85-90% pure). Crystalized from heptane/ethyl acetate 2:1 at rt. A pink solid, was obtained with sufficient purity (>97%). (550 mg, 1.91 mmol, 14%); $^1$H NMR (600 MHz, MeOD) $\delta$ 10.34 (s, 1H), 8.57 (d, *J* = 8.1 Hz, 2H), 7.57 (d, *J* = 8.1 Hz, 2H), 4.39 (s, 2H), 1.51 (s, 9H); $^{13}$C NMR (151 MHz, MeOD) $\delta$ 167.6, 159.2, 158.6, 146.5, 132.1, 129.2, 128.9, 80.3, 44.7, 28.7.

**(4-(1,2,4,5-Tetrazin-3-yl)phenyl)methanaminium trifluoroacetate (Tetrazine 3):**

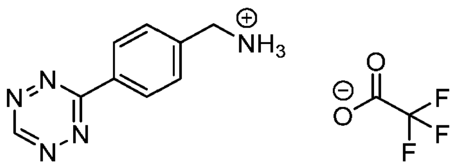

*tert*-Butyl (4-(1,2,4,5-Tetrazin-3-yl)benzyl)carbamate (338 mg, 1.17 mmol, 1 eq.) was dissolved in 50 mL DCM. Afterwards 20 mL of TFA were added and the reaction was left to stir for 30 minutes at r.t. The solvent was removed under reduced pressure resulting to a pink solid **(Tetrazine 3)** (350 mg, 1.16 mmol, 99%) which was used at the next step without further purification. $^1$H NMR (600 MHz, MeOD) $\delta$ 10.39 (s, 1H), 8.69 (d, *J* = 8.4 Hz, 2H), 7.76 (d, *J* = 8.4 Hz, 2H), 4.30 (s, 2H);$^{13}$C NMR (151 MHz, MeOD) $\delta$ 167.3, 162.85 (q), 159.5, 139.2, 134.2, 130.8, 129.7, 118.14 (q), 43.9.

Tetrazine 4 (as shown in Figure 3) was prepared as follows:

**5-((4-(1,2,4,5-Tetrazin-3-yl)benzyl)amino)-5-oxopentanoic acid**

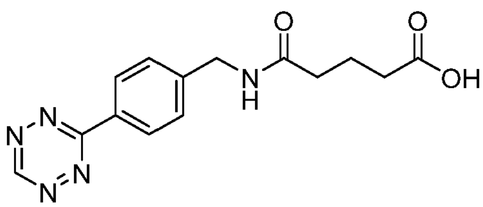

(4-(1,2,4,5-Tetrazin-3-yl)phenyl)methanaminium trifluoroacetate (tetrazine 3, 210 mg, 0.69 mmol, 1 eq.) and glutaric anhydride (79.5 mg, 0.69 mmol, 1 eq., purified directly before use) were dissolved in a 100 mL flask then diluted with 20 mL DCM. After that, $Et_3N$ (194 µL, 1.39 mmol, 2 eq.) was added, and the reaction was stirred at r.t for 1.5 h. Volatiles

were removed under vacuum and the concentrated reddish solid was washed with a solution 3:1 heptane: EtOAc. The crude was purified by flash chromatography (DCM:MeOH 9:1 0.2% Acetic acid), which yielded the desired compound as a pink solid (160mg, 0.53 mmol, 76%). $^{1}H$ NMR (600 MHz, MeOD) $\delta$ 10.34 (s, 1H), 8.58 (d, $J$ = 8.4 Hz, 2H), 7.59 (d, $J$ = 8.3 Hz, 2H), 4.53 (s, 2H), 2.38 (td, $J$ = 7.4, 2.9 Hz, 4H), 1.97 (p, $J$ = 7.4 Hz, 2H). $^{13}C$ NMR (151 MHz, MeOD) $\delta$ 176.8, 175.4, 167.6, 159.2, 145.6, 132.3, 129.3, 129.3, 43.7, 36.0, 34.1, 22.3.

***tert*-Butyl (37,41-dioxo-41-((4-(1,2,4,5-tetrazin-3-yl)benzyl)amino)- 3,6,9,12,15,18,21,24,27,30,33-undecaoxa-36-azahentetracontyl)carbamate**

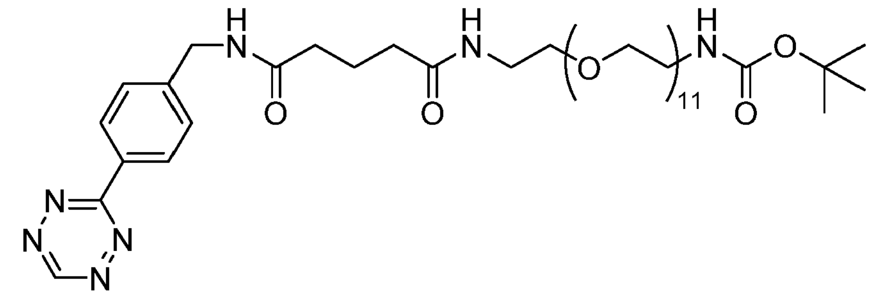

$Et_3N$ (70 µL, 0.50 mmol, 1.5 eq.) was added to a stirred mixture of 5-((4-(1,2,4,5-Tetrazin-3-yl)benzyl)amino)-5-oxopentanoic acid (100 mg, 0.33 mmol, 1 eq.), O-(2-aminoethyl)-O'-[2-(Boc-amino)ethyl]decaethylene glycol (214 mg, 0.33 mmol, 1 eq.) and HATU (158 mg, 0.42 mmol, 1.25 eq.) in dry DMF (5 mL). After stirring for 2 hours at room temperature, the mixture was diluted in water (10 mL) and extracted afterwards with DCM (3x 20 mL). The organic layers were combined was dried over $MgSO_4$, filtered and concentrated under reduced pressure. The resulting residue was purified by column chromatography on silica using MeOH/DCM mixture (6/94 v/v) as eluent, giving the desired product as a red solid (265 mg, 0.29 mmol, 86%); $^{1}H$ NMR (400 MHz, MeOD) $\delta$ 10.33 (s, 1H), 8.60 - 8.51 (m, 2H), 7.63 - 7.51 (m, 2H), 4.50 (s, 2H), 3.78 - 3.57 (m, 40H), 3.54 (t, $J$ = 5.5 Hz, 2H), 3.50 (t, $J$ = 5.6 Hz, 2H), 3.36 (t, $J$ = 5.5 Hz, 2H), 3.25 - 3.17 (m, 2H), 2.33 (t, $J$ = 7.5 Hz, 2H), 2.27 (t, $J$ = 7.4 Hz, 2H), 2.00 - 1.91 (m, 2H), 1.44 (s, 9H).

**37,41-dioxo-41-((4-(1,2,4,5-Tetrazin-3-yl)benzyl)amino)-3,6,9,12,15,18,21,24,27,30,33-undecaoxa-36-azahentetracontan-1-aminium trifluoroacetate (tetrazine 4)**

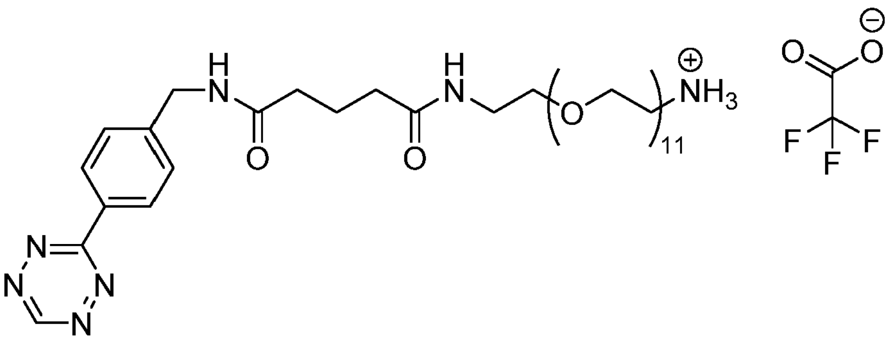

tert-Butyl (37,41-dioxo-41-((4-(1,2,4,5-tetrazin-3-yl)benzyl)amino)-3,6,9,12,15,18,21,24,27,30,33-undecaoxa-36-azahentetracontyl)carbamate (135 mg, 0.146 mmol) was mixed with 10 mL TFA and 35 mL dry DCM. After stirring for 30 min at room temperature, all volatiles were removed in vacuo, and the crude was dried on high vacuum, which yielded a dark red solid (137 mg, 0.136 mmol, 93%). $^{1}H$ NMR (400 MHz, MeOD) $\delta$ 10.33 (s, 1H), 8.56 (d, $J$ = 8.3 Hz, 2H), 7.57 (d, $J$ = 8.2 Hz, 2H), 4.50 (s, 2H), 3.80 - 3.75 (m, 2H), 3.74 - 3.58 (m, 40H), 3.54 (t, $J$ = 5.5 Hz, 2H), 3.36 (t, $J$ = 5.6 Hz, 2H), 3.18 (t, $J$ = 5.1 Hz, 2H), 2.33 (t, $J$ = 7.5 Hz, 2H), 2.27 (t, $J$ = 7.5 Hz, 2H), 1.95 (p, $J$ = 7.4 Hz, 2H).

Tetrazine 5 (as shown in Figure 3) was prepared as follows:

***tert*-butyl (8-((6-cyanopyridin-3-yl)amino)-8-oxooctyl)carbamate**

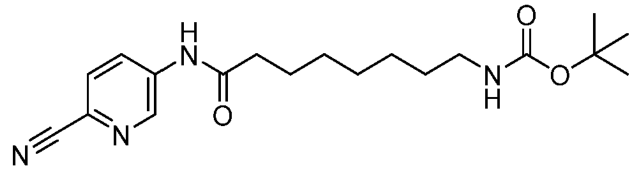

5-amino-2-cyanopyridine (1.0 g, 9.38 mmol, 1.0 eq.) and 8-((*tert*-butoxycarbonyl)amino)octanoic acid (2.39 g, 9.23, 1.1 eq.) were dissolved in dry DMF (15 mL), followed by the addition of $Et_3N$ (2.34 mL, 16.79 mmol, 2.0 eq.). HATU (3.83 g, 10.07 mmol, 1.2 eq.) was added in one portion and the reaction was allowed to stir for 17 hours at room temperature. The reaction was quenched with sat. $Na_2CO_3$ and the crude was extracted with 3x DCM. The product was purified by column chromatography, yielding the desired product (2.06 g, 5.72 mmol, 61%); $^1H$ NMR (400 MHz, $CDCl_3$) $\delta$ 8.96 (s, 1H), 8.72 (d, *J* = 2.5 Hz, 1H), 8.44 (dd, *J* = 8.6, 2.6 Hz, 1H), 7.64 (d, *J* = 8.5 Hz, 1H), 4.65 (s, 1H), 3.08 (q, *J* = 6.5 Hz, 2H), 2.42 (t, *J* = 7.5 Hz, 2H), 1.72 (q, *J* = 7.2 Hz, 2H), 1.43 (s, 9H), 1.38 - 1.21 (m, 8H); $^{13}C$ NMR (101 MHz, $CDCl_3$) $\delta$ 173.0, 156.5, 142.1, 138.3, 129.2, 127.4, 126.2, 117.6, 79.5, 40.4, 37.3, 33.2, 29.9, 28.6, 26.3, 25.0.

***tert*-butyl (8-oxo-8-((6-(6-(pyridin-2-yl)-1,4-dihydro-1,2,4,5-tetrazin-3-yl)pyridin-3-yl)amino)octyl)carbamate:**

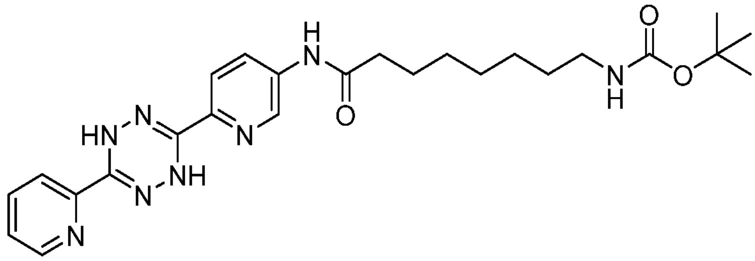

*tert*-butyl (8-((6-cyanopyridin-3-yl)amino)-8-oxooctyl)carbamate (500 mg, 1.39 mmol, 1.0 eq.) and 2-cyanopyridine (578 mg, 5.55 mmol, 4.0 eq.) were dissolved in EtOH (abs) (1.5mL), followed by the addition of hydrazine hydrate (1.35 mL). The reaction was heated to 90°C in a closed $\mu$W-vial, under $N_2$-atmosphere and stirred for overnight. The reaction was concentrated, suspended in $H_2O$ and the crude was extracted with 3x DCM. The combined fractions were purified by column chromatography, yielding the desired product as a yellow solid (362 mg, 0.74 mmol, 53%); $^1H$ NMR (400 MHz, $CDCl_3$) $\delta$ 8.63 (d, *J* = 2.5 Hz, 1H), 8.60 - 8.54 (m, 1H), 8.53 (s, 1H), 8.48 (s, 1H), 8.19 (dd, *J* = 8.7, 2.5 Hz, 1H), 8.08 - 7.97 (m, 2H), 7.75 (td, *J* = 7.8, 1.7 Hz, 1H), 7.34 (ddd, *J* = 7.5, 4.9, 1.2 Hz, 1H), 4.54 (s, 1H), 3.11 (q, *J* = 6.9 Hz, 2H), 2.40 (t, *J* = 7.4 Hz, 2H), 1.74 (p, *J* = 7.2 Hz, 2H), 1.53 - 1.45 (m, 1H), 1.44 (s, 9H), 1.40 - 1.25 (m, 6H), 1.28 - 1.22 (m, 1H), 1.14 (dt, *J* = 25.7, 7.1 Hz, 1H).$^{13}C$ NMR (151 MHz, $CDCl_3$) $\delta$ 172.4, 156.3, 148.5, 147.7, 146.9, 146.5, 142.7, 139.3, 137.8, 136.4, 127.4, 125.0, 121.7, 121.4, 79.3, 40.2, 33.2, 32.0, 26.8, 26.3, 25.5, 25.2, 22.8.

***tert*-butyl (8-oxo-8-((6-(6-(pyridin-2-yl)-1,2,4,5-tetrazin-3-yl)pyridin-3-yl)amino)octyl)carbamate:**

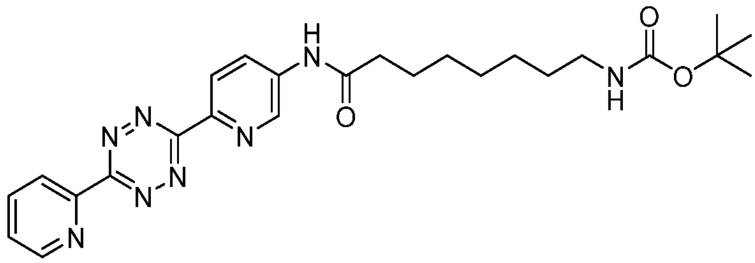

*tert-butyl* (8-oxo-8-((6-(6-(pyridin-2-yl)-1,4-dihydro-1,2,4,5-tetrazin-3-yl)pyridin-3-yl)amino)octyl) carbamate (60 mg, 0.12 mmol, 1.0 eq.) was dissolved in dry DCM (6 mL) and cooled to 0°C, followed by the portion wise addition of PIDA (47 mg, 0.15 mmol, 1.2 eq.). The reaction was allowed to warm to room temperature and was stirred for 3h. Celite was added to the mixture and the mixture was concentrated. The crude was purified by column chromatography, which yielded a red solid (43 mg, 0.09 mmol, 72%). $^1$H NMR (600 MHz, $CDCl_3$) δ 8.96 (dd, *J* = 4.8, 1.6 Hz, 1H), 8.90 (d, *J* = 2.5 Hz, 1H), 8.74 (t, *J* = 8.9 Hz, 2H), 8.65 (dd, *J* = 8.7, 2.5 Hz, 1H), 8.56 (s, 1H), 8.00 (td, *J* = 7.8, 1.8 Hz, 1H), 7.57 (ddd, *J* = 7.6, 4.7, 1.2 Hz, 1H), 4.58 (s, 1H), 3.12 (q, *J* = 6.8 Hz, 2H), 2.46 (t, *J* = 7.4 Hz, 2H), 1.76 (q, *J* = 7.3 Hz, 2H), 1.50 - 1.46 (m, 2H), 1.44 (s, 9H), 1.41 - 1.30 (m, 6H). $^{13}$C NMR (151 MHz, $CDCl_3$) δ 172.7, 163.7, 163.5, 156.4, 151.1, 150.3, 144.5, 141.9, 138.3, 137.6, 127.1, 126.6, 125.4, 124.5, 79.4, 40.3, 37.4, 29.9, 28.8, 28.6, 28.5, 26.2, 25.1.

**8-amino-*N*-(6-(6-(pyridin-2-yl)-1,2,4,5-tetrazin-3-yl)pyridin-3-yl)octanamide 2,2,2-trifluoroacetate (Tetrazine 5)**

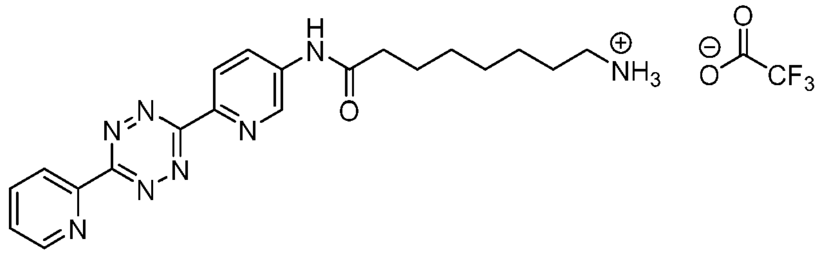

*tert*-butyl (8-oxo-8-((6-(6-(pyridin-2-yl)-1,2,4,5-tetrazin-3-yl)pyridin-3-yl)amino)octyl)carbamate (25mg, 0.05 mmol, 1.0 eq.) was dissolved in dry DCM 2 mL), to which was added dropwise TFA (49 μL, 0.51 mmol, 10.0 eq.). The reaction was stirred for 2 h at room temperature, after which all volatiles were removed in vacuo and the crude was dried on high vacuum, which yielded of a dark red solid (19 mg, 0.048 mmol, 95%) $^1$H NMR (600 MHz, MeOD) δ 9.10 (d, *J* = 2.5 Hz, 1H), 8.93 (ddd, *J* = 5.0, 1.7, 0.9 Hz, 1H), 8.86 (dt, *J* = 8.0, 1.1 Hz, 1H), 8.80 (d, *J* = 8.7 Hz, 1H), 8.49 (dd, *J* = 8.7, 2.5 Hz, 1H), 8.31 (tt, *J* = 7.8, 1.3 Hz, 1H), 7.85 (ddt, *J* = 7.5, 4.9, 1.1 Hz, 1H), 2.93 (t, J = 7.7 Hz, 2H), 2.51 (t, J = 7.4 Hz, 2H), 1.76 (p, J = 7.5 Hz, 2H), 1.68 (p, J = 7.4 Hz, 2H), 1.48 - 1.41 (m, 6H). $^{13}$C NMR (151 MHz, MeOD) δ 175.3, 164.3, 160.6, 160.4, 160.1, 159.8, 150.6, 142.3, 128.9, 126.5, 125.9, 117.7, 115.8, 40.7, 37.8, 30.0, 29.9, 28.5, 27.3, 26.3.

Tetrazine 6 (as shown in Figure 3) was prepared as follows:

**6-(6-(Pyridin-2-yl)-1,4-dihydro-1,2,4,5-tetrazin-3-yl)pyridin-3-amine:**

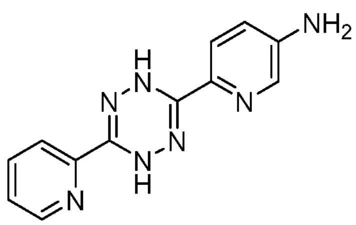

5-amino-2-cyanopyridine (1.140 g, 9.60 mmol, 1 eq.), 2-cyanopyridine (0.995 g, 9.60 mmol, 1 eq.) and hydrazine monohydrate (2.35 mL, 48 mmol, 5 eq.) were added in a sealed vial at room temperature. The vial was heated at 90 °C and the resulting solution was stirred for 14 h at this temperature. After cooling to room temperature, the resulting orange solid was washed with cold water, dried and purified by column chromatography using a gradient elution (EtOAc-heptane, 1:1 to 6:1) to furnish the desired compound (532 mg, 2.10 mmol, 22%) as a pale orange solid; $^1$H NMR (600 MHz, DMSO) $\delta$ 8.71 (s, 1H), 8.65 (s, 1H), 8.64 - 8.60 (m, 1H), 7.98 - 7.87 (m, 3H), 7.65 (d, *J* = 8.6 Hz, 1H), 7.51 (ddd, *J* = 7.4, 4.8, 1.3 Hz, 1H), 7.00 (dd, *J* = 8.6, 2.7 Hz, 1H), 5.87 (s, 2H); $^{13}$C NMR (151 MHz, DMSO) $\delta$ 148.5, 147.5, 146.7, 146.6, 146.6, 137.34, 134.1, 134.1, 125.1, 121.8, 120.8, 120.3.

**6-(6-(Pyridin-2-yl)-1,2,4,5-tetrazin-3-yl)pyridin-3-amine:**

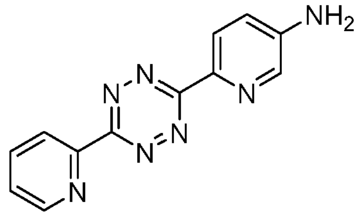

6-(6-(Pyridin-2-yl)-1,4-dihydro-1,2,4,5-tetrazin-3-yl)pyridin-3-amine (532 mg, 2.09 mmol, 1 eq.) was dissolved in DCM (50 mL) and PIDA (809.8 mg, 2.51 mmol, 1.2 eq.) was added in one portion at room temperature. The resulting solution was stirred at this temperature for 2 h. Volatiles were removed under reduced pressure and the resulting crude material was purified by column chromatography using a gradient elution (DCM-MeOH, 99:1 to 9:1) to afford the desired compound (263 mg, 1.04 mmol, 50%) as a dark red solid; $^1$H NMR (600 MHz, DMSO) $\delta$ 8.91 - 8.88 (m, 1H), 8.53 (d, $J$ = 7.9 Hz, 1H), 8.36 (d, $J$ = 8.6 Hz, 1H), 8.24 (d, $J$ = 2.7 Hz, 1H), 8.12 (td, $J$ = 7.7, 1.8 Hz, 1H), 7.68 (ddd, $J$ = 7.6, 4.7, 1.2 Hz, 1H), 7.13 (dd, $J$ = 8.6, 2.7 Hz, 1H), 6.36 (s, 2H); $^{13}$C NMR (151 MHz, DMSO) $\delta$ 162.9, 162.5, 150.4, 147.9, 137.6, 137.3, 136.0, 126.2, 125.7, 123.7, 118.9.

**5-oxo-5-((6-(6-(pyridin-2-yl)-1,2,4,5-tetrazin-3-yl)pyridin-3-yl)amino)pentanoic acid:**

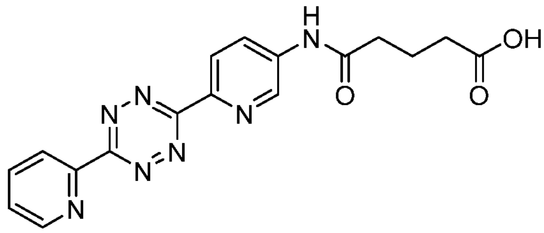

A solution of 6-(6-(Pyridin-2-yl)-1,2,4,5-tetrazin-3-yl)pyridin-3-amine (275 mg, 1.094 mmol, 1 eq.) and glutaric anhydride (624 mg, 5.46mmol, 5eq.) in dry THF (20 mL) was heated at 110°C for 3 days in a sealed flask. After cooling, volatiles were removed under vacuum and the precipitate was washed with DCM (2×12 mL) and EtOAc (12 mL) to yield the desired compound as a red solid (333 mg, 0.911 mmol, 83%); $^1$H NMR (600 MHz, DMSO) $\delta$ 12.11 (s, 1H), 10.57 (s, 1H), 9.04 (d, $J$ = 2.5 Hz, 1H), 8.93 (d, $J$ = 4.4 Hz, 1H), 8.61 (d, $J$ = 8.7 Hz, 1H), 8.59 (d, $J$ = 7.8 Hz, 1H), 8.42 (dd, $J$ = 8.7, 2.5 Hz, 1H), 8.15 (td, $J$ = 7.7, 1.8 Hz, 1H), 7.72 (ddd, $J$ = 7.6, 4.7, 1.2 Hz, 1H), 2.48 (d, $J$ = 7.4 Hz, 2H), 2.32 (t, $J$ = 7.3 Hz, 2H), 1.86 (p, $J$ = 7.4 Hz, 2H); $^{13}$C NMR (151 MHz, DMSO) $\delta$ 174.1, 172.0, 163.0, 162.7, 150.6, 150.2, 143.8, 141.3, 138.4, 137.8, 126.5, 126.1, 124.9, 124.2, 35.4, 32.9, 20.1.

***tert*-Butyl (37,41-dioxo-41-((6-(6-(pyridin-2-yl)-1,2,4,5-tetrazin-3-yl)pyridin-3-yl)amino)-3,6,9,12,15,18, 21,24,27,30,33-undecaoxa-36-azahentetracontyl)carbamate:**

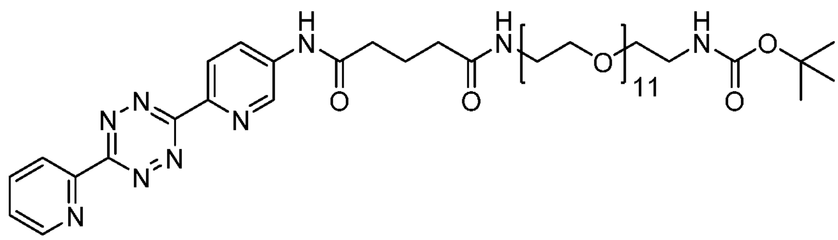

$Et_3N$ (71 μL, 0.466 mmol, 1.5 eq.) was added to a stirred mixture of 5-oxo-5-((6-(6-(pyridin-2-yl)-1,2,4,5-tetrazin-3-yl)pyridin-3-yl)amino)pentanoic acid (114 mg, 0.3115 mmol, 1 eq.), O-(2-Aminoethyl)-O'-[2-(Boc-amino) ethyl]decaethylene glycol (249 mg, 0.373 mmol, 1.2 eq.) and HATU (129 mg, 0.389 mmol, 1.25 eq.) in DMF (8 mL). After stirring overnight at rt, the mixture was diluted in water (20 mL) and extracted afterwards with DCM (40 mL) three times. The organic phases were combined was dried over $MgSO_4$, filtered and concentrated under reduced pressure. The resulting residue was purified by column chromatography on silica using a gradient of MeOH in DCM (0-10%) giving the

desired compound as a purple/red solid (157mg, 0.158 mmol, 50%); $^1$H NMR (600 MHz, $CDCl_3$) $\delta$ 9.61 (s, 1H), 9.04 (s, 1H), 8.98 (d, *J* = 6.6 Hz, 1H), 8.74 (d, *J* = 2.3 Hz, 1H), 8.73 (d, *J* = 1.8 Hz, 1H), 8.65 (dd, *J* = 8.6, 2.5 Hz, 1H), 8.01 (td, *J* = 7.8, 1.8 Hz, 1H), 7.58 (ddd, *J* = 7.6, 4.7, 1.2 Hz, 1H), 6.59 (s, 1H), 5.05 (s, 1H), 3.76 - 3.56 (m, 42H), 3.53 (t, *J* = 5.2 Hz, 2H), 3.47 (q, *J* = 5.2 Hz, 2H), 3.30 (d, *J* = 5.9 Hz, 2H), 2.58 (t, *J* = 6.9 Hz, 2H), 2.38 (t, *J* = 6.8 Hz, 2H), 2.10 (p, *J* = 6.4 Hz, 2H), 1.43 (s, 9H); $^{13}$C NMR (151 MHz, $CDCl_3$) $\delta$ 173.3, 172.7, 163.6, 163.5, 151.0, 150.4, 144.0, 142.2, 138.7, 137.6, 126.6, 126.5, 125.3, 124.4, 70.6, 70.3, 70.2, 40.5, 39.4, 36.2, 35.1, 28.5, 21.6.

**37,41-dioxo-41-((6-(6-(pyridin-2-yl)-1,2,4,5-tetrazin-3-yl)pyridin-3-yl)amino)-3,6,9,12,15,18,21,24,27,30,33-undecaoxa-36-azahentetracontan-1-aminium trifluoroacetate (Tetrazine 6):**

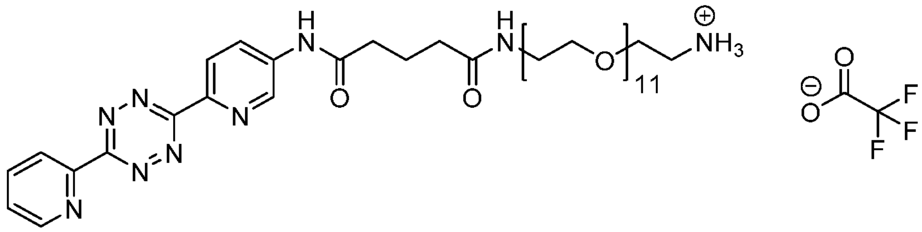

*tert-Butyl* (37,41-dioxo-41-((6-(6-(pyridin-2-yl)-1,2,4,5-tetrazin-3-yl)pyridin-3-yl)amino)-3,6,9,12,15,18,21,24,27,30,33-undecaoxa-36-azahentetracontyl)carbamate (157 mg, 0.158 mmol, 1 eq.) was dissolved in 25mL dry DCM together with 10 mL TFA and left to react for 30 min at rt, resulting in complete conversion to Tetrazine 6 (159 mg, 0.158 mmol). $^1$H NMR (600 MHz, MeOD) $\delta$ 9.06 (d, *J* = 2.4 Hz, 1H), 8.88 (d, *J* = 4.0 Hz, 1H), 8.76 (t, *J* = 8.1, 7.3 Hz, 2H), 8.48 (dd, *J* = 8.7, 2.3 Hz, 1H), 8.17 (td, *J* = 7.8, 1.8 Hz, 1H), 7.73 (ddd, *J* = 7.6, 4.8, 1.2 Hz, 1H), 3.79 - 3.72 (m, 2H), 3.72 - 3.60 (m, 42H), 3.56 (t, *J* = 5.5 Hz, 2H), 3.39 (t, *J* = 5.5 Hz, 2H), 3.20 - 3.17 (m, 2H), 2.54 (t, *J* = 7.3 Hz, 2H), 2.34 (t, *J* = 7.4 Hz, 2H), 2.04 (p, *J* = 7.3 Hz, 2H);$^{13}$C NMR (151 MHz, MeOD) $\delta$ 175.4, 174.3, 164.7, 164.5, 162.9 (q), 151.5, 151.3, 145.3, 142.7, 140.3, 139.5, 128.3, 128.2, 126.1, 125.6, 118.2 (q), 67.9, 40.6, 40.3, 36.8, 36.0, 22.6.

**Example 2: Preparation of radiolabeled tetrazine**

Tetrazine 7 (as shown in Figure 4) was prepared as described in (Rossin, R.; Verkerk, P. R.; van den Bosch, S. M.; Vulders, R. C. M.; Verel, I.; Lub, J.; Robillard, M. S., In Vivo Chemistry for Pretargeted Tumor Imaging in Live Mice. Angew Chem Int Edit 2010, 49 (19), 3375-3378.)

$^{111}$In-Labeled tetrazine 8 ([$^{111}$In]**8**, as shown in Figure 4) was prepared by radiolabeling of tetrazine 7. For the radiolabeling, [$^{111}$In]$InCl_3$ (50-100 $\mu$L, 10-20 MBq) was added to a solution of 1 M $NH_4OAc$ pH 5.0 (10-20 $\mu$L) and DOTA-Tz precursor **19** (4-6 $\mu$g, 2-3 $\mu$L from stock solution in metal-free water). Depending on the amount of [$^{111}$In]$InCl_3$ used for the labeling, one fifth of the volume 1 M $NH_4OAc$ pH 5.0 was needed. The mixture was heated at 60 °C for 5 min, before 13 mM gentisic acid in saline (2 $\mu$L) and 10 mM DTPA in PBS (3 $\mu$L) were added. The mixture was heated at 60 °C for 5 additional min. Analysis was performed by radio-HPLC on a Aeris Peptide 3.6 $\mu$m XB-C18 column (150 $\times$ 4.6 mm) using a gradient of acetonitrile ($CH_3CN$) in water with 0.1% TFA. Gradient conditions: 0-1 min - 5% $CH_3CN$, 1-8 min - linear increase of $CH_3CN$ content to 75%, 8-9 min - 75% $CH_3CN$, 9-9.5 min - linear decrease of $CH_3CN$ content to 5%, 9.5-10 min - 5% $CH_3CN$, elution speed 1.5 mL/min. [$^{111}$In]**8** was afforded in a radiochemical yield (RCY) of 94$\pm$5% *(n* = 7). Example of HPLC radiochromatogram of [$^{111}$In]**8** is shown in Figure 5.

**Example 3: Preparation of TCO-decorated polymer and TCO-decorated monoclonal antibody TCO-CC49**

TCO-decorated monoclonal antibody TCO-CC49 (corresponding to Structure 1 in Figure 6) was prepared as described in (Rossin, R.; Van Duijnhoven, S. M. J.; Läppchen, T.; Van Den Bosch, S. M.; Robillard, M. S. Trans-Cyclooctene Tag with Improved Properties for Tumor Pretargeting with the Diels-Alder Reaction. Mol. Pharm. 2014, 11 (9), 3090-3096.). In this publication, TCO-CC49 is referred to as CC49-TCO(2).

Namely, CC49 monoclonal antibody was produced from the CC49 hybridoma cell line acquired from the American Type Culture Collection (ATCC) as described in (Rossin, R.; Verkerk, P. R.; van den Bosch, S. M.; Vulders, R. C. M.; Verel, I.; Lub, J.; Robillard, M. S., In Vivo Chemistry for Pretargeted Tumor Imaging in Live Mice. Angew Chem Int Edit 2010, 49 (19), 3375-3378.) and modified with axial (E)-2,5-dioxopyrrolidin-1-yl 2-(cyclooct-4-en-1-yloxy)acetate (structure shown below) as described in the same publication.

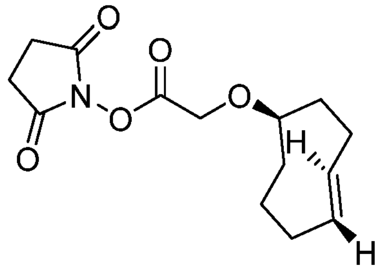

Axial (E)-2,5-dioxopyrrolidin-1-yl 2-(cyclooct-4-en-1-yloxy)acetate was prepared as described in (Rossin, R.; van den Bosch, S. M.; ten Hoeve, W.; Carvelli, M.; Versteegen, R. M.; Lub, J.; Robillard, M. S. Highly Reactive Trans -Cyclooctene Tags with Improved Stability for Diels-Alder Chemistry in Living Systems. Bioconjug. Chem. 2013, 24 (7), 1210-1217.) In this publication, axial (E)-2,5-dioxopyrrolidin-1-yl 2-(cyclooct-4-en-1-yloxy)acetate is referred to as compound 7b.

TCO-decorated polymer TCO-KS254 (corresponding to Structure 2 in Figure 6) was prepared as described in (Stéen, E. J. L.; Jørgensen, J. T.; Johann, K.; Nørregaard, K.; Sohr, B.; Svatunek, D.; Birke, A.; Shalgunov, V.; Edem, P. E.; Rossin, R.; et al. Trans -Cyclooctene-Functionalized PeptoBrushes with Improved Reaction Kinetics of the Tetrazine Ligation for Pretargeted Nuclear Imaging. ACS Nano 2020, 14 (1), 568-584.) In this publication, TCO-KS254 is referred to as Peptobrush 1.

**Example 4: preparation of Tz-agarose**

Amino-tetrazine **(1-6)** was dissolved in phosphate-buffered saline (PBS, 120 mM NaCl, 10 mM phosphate buffer pH 7.4) to a concentration of 4 mM (tetrazine **5** could only be dissolved to a concentration of 2.7 mM). This solution was added to dry NHS-activated agarose resin (Pierce/Thermo Fisher Scientific) at the ratio of 1 mL solution per 60 mg resin. The resin was shaken in a plastic tube with a filter frit for 2-3 h at room temperature, then tetrazine solution was removed by vacuum filtration, and the resin was washed 3 times with PBS at the same mL-per-mg resin ratio. After that, Tris-HCl buffer (0.5M, pH 7.4) was added to the resin, the resin was shaken for 2-3 h at room temperature or overnight at 4°C and washed 3 times with PBS. Control resin (tetrazine-free quenched agarose) was prepared in the same way, but pure PBS without amino-tetrazines was used for the initial incubation.

Aliquots (50 μL or more) were withdrawn from each amino-tetrazine solution before incubation with the resin. At the end of incubation, when solutions were removed by vacuum filtration, a second set of aliquots were withdrawn from each solution. All aliquots were analyzed by HPLC, and areas of absorption peaks at 254 nm were measured. Examples of HPLC chromatograms for amino-tetrazines **1-6** are shown in Figure 7.

HPLC conditions: Luna C18 5μm 150x4.6 mm, eluted at 1.5 mL/min with a gradient of acetonitrile ($CH_3CN$) in water with 0.1% trifluoroacetic acid (TFA) in both solvents. Injection volume was 10 μL for all samples. Gradient conditions: 0-1 min - 5% $CH_3CN$, 1-8 min - linear increase of $CH_3CN$ content to 75%, 8-9 min - 75% $CH_3CN$, 9-9.5 min - linear decrease of $CH_3CN$ content to 5%, 9.5-10 min - 5% $CH_3CN$.

Coupling efficiency (CE) for each tetrazine-agarose batch was calculated according to the formula:

$$\%CE = 100\% \times (Abs_{before} / Abs_{after}),$$

where $Abs_{before}$ and $Abs_{after}$ are areas of the corresponding tetrazine peaks on HPLC chromatograms obtained from amino-tetrazine solutions respectively before and after incubation with the resin.

The calculated efficiency of the coupling of the six amino-tetrazine derivatives to the agarose resin is shown in Table 1.

Table 1. Coupling efficiency for tetrazine-decorated agarose

| **Tetrazine** | **%coupled, mean±SD** |
|---|---|
| 1 | 90±5 |
| 2 | 80±0 |
| 3 | 92±0 |
| 4 | 17±1 |
| 5 | 83±2 |

(continued)

| Tetrazine | %coupled, mean±SD |
|---|---|
| 6 | 21±1 |
| n=2 for all experiments | |

As evident from the data in Table 1, the coupling to agarose of all tetrazines except tetrazine 4 and tetrazine 6 proceeded with high efficiency (at least 80%). Tetrazine 4 and tetrazine 6 were coupled to agarose with lower efficiencies (17-21%), but obtained loadings are nevertheless suitable for trapping experiments (Example 6).

**Example 5: column filling**

Tetrazine-conjugated agarose, prepared as described above, was transferred into a double-fritted filtration column (Biotage) and packed between two frits. After swelling in PBS, the resin occupied the volume of 0.5 mL per 60 mg dry weight.

**Example 6: Trapping experiments with TCO-CC49 and TCO-KS254 solutions in PBS**

Tetrazine-agarose columns, two for each tetrazine, each prepared from 33 mg of dry NHS-agarose as described above (bed volume approx. 0.25 mL), and a control column prepared from tetrazine-free agarose, were equilibrated with 2-3 bed volumes of PBS. Antibody TCO-CC49 (approx. 1 TCO groups per 20 kDa molecular weight) or polymer TCO-KS254 (approx. 1 TCO group per 5 kDa molecular weight) were dissolved in PBS with to a mass concentration of 100 μg/mL. Aliquots of these solutions (0.4 mL) were pumped through the tetrazine-agarose columns at 0.5 mL/min pumping speed using an infusion pump (AL-1000, World Precision Instruments). When all solution had entered the resin bed, an aliquot of PBS (0.4 mL) was immediately added on top of it and pumped through the column at 0.5 mL/min. Total eluate (total volume approx. 0.8 mL) was collected and the amount of TCO-CC49 or TCO-KS254 was determined by $^{111}$In-tetrazine ([$^{111}$In]8) titration.

For each column, trapping of both TCO-CC49 and TCO-KS254 was tested, and the column was rinsed with 2-3 volumes of PBS between tests. For pairs of columns with the same tetrazine, the order of testing (TCO-CC49 first or TCO-KS254 first) for one column was opposite to the order of testing for another column.

An aliquot of each trapping column eluate (6 μL) was mixed with an equal volume of 10 μM [$^{111}$In]**8** solution. Resulting mixtures were shaken at 37°C for at least 1 hour to ensure full consumption of reactive TCO moieties. Then NuPage LDS Sample buffer was added to all mixtures according to the supplier's instructions, samples were heated for 10 min at 70°C and applied on the NuPAGE 4-12% Bis-Tris SDS-PAGE gels. SDS-PAGE was run in MES-SDS buffer at 150V for 40-45 min. Separation was monitored by running SeeBlue® Plus2 Pre-Stained Protein Standard along with radiolabeled polymer samples. Control mixtures of 6 μL of [$^{111}$In]**8** solution with 6 μL of PBS and reference mixtures of 6 μL of [$^{111}$In]**8** solution and 6 μL of 50 μg/mL solutions of TCO-CC49 or TCO-KS254 were prepared and processed in the same manner. Developed gels were exposed against phosphor storage screens (PerkinElmer Multisensitive), which were then read in the Cyclone Plus phosphorimager (Packard Instruments, USA). Autoradiograms were quantified using Optiquant 3.0 software (Packard Instruments). Examples of obtained SDS-PAGE gel autoradiograms are shown in Figure 8. Molecular weight reference ladder from the photo of the same gel aligned and merged to the autoradiogram is shown to the left in Figure 8. Radioactivity found in the section of the gel corresponding to molecular weights of 60 kDa and above (using SeeBlue standards as reference) was considered polymer-bound or antibody-bound.

Trapping efficiency (TE) was calculated according to the formula:

$$\%TE = 100\% \times (1 - \%B_{Tz} / \%B_{noTz}),$$

where $\%B_{Tz}$ is the percentage of polymer/antibody bound $^{111}$In activity in the eluate collected from a column with tetrazine-conjugated agarose, while $\%B_{noTz}$ is the percentage of polymer/antibody bound $^{111}$In activity in the eluate collected from a control column with tetrazine-free agarose.

For each column, evaluation of trapping efficiency for TCO-CC49 and TCO-KS254 was repeated 9 days after the first evaluation. In between evaluations, the columns were stored at 4°C in phosphate-buffered saline with 0.1% vol/vol of ProClin™ 150 (Sigma-Aldrich).

The trapping efficiencies of agarose resins decorated with the six amino-tetrazine derivatives for each of the TCO-derivatives TCO-CC49 and TCO-KS254, respectively, as determined by $^{111}$In tetrazine titration in the first and second evaluations, are shown in Figure 9 and in Table 2:

Table 2. Trapping efficiency for tetrazine-decorated agarose

| Tetra -zine | %TE mean±SD 1st evaluation TCO-CC49 | %TE mean±SD 2nd evaluation TCO-CC49 | %TE mean±SD 1st evaluation TCO-KS254 | %TE mean±SD 2nd evaluation TCO-KS254 |
|---|---|---|---|---|
| 1 | 99±0.4 | 89±6 | 80±10 | 60±1 |
| 2 | 98±0.4 | 92±0.4 | 55±11 | 43±1 |
| 3 | 99±1 | 96±0.4 | 93±10 | 78±1 |
| 4 | 96±6 | 92±0.4 | 75±0.2 | 56±2 |
| 5 | 98±0.4 | 89±3 | 85±5 | 67±0.3 |
| 6 | 93±7 | 72±1 | 64±0 | 50±6 |
| n=2 for all experiments | | | | |

As evident from the data in Figure 9, for all amino-tetrazine derivatives, agarose resins decorated with the corresponding tetrazines trapped 70% or more of TCO-CC49 and 40% or more of TCO-KS254 on a single pass. Trapping efficiencies measured during the second evaluation were only slightly lower than during the first evaluation. Thus, all amino-tetrazine tetrazines, when conjugated to agarose resin, demonstrated efficient removal of TCO-modified compounds from the solution passed through the resin. Two surprising trends were observed in the experimental results. First, trapping efficiencies for methyl-substituted tetrazines 1 and 2 were not significantly lower than for unsubstituted tetrazines 3 and 4 and (bis)pyridyl-substituted tetrazines 5 and 6, even though the kinetics of IEDDA reaction are known to be at least an order of magnitude slower for methyl-substituted tetrazines than for unsubstituted and (bis)pyridyl-substituted analogs (Oliveira, B. L.; Guo, Z.; Bernardes, G. J. L. Inverse Electron Demand Diels-Alder Reactions in Chemical Biology. Chem. Soc. Rev. 2017, 46 (16), 4895-4950; Stéen, E. J. L.; Jørgensen, J. T.; Denk, C.; Battisti, U. M.; Nørregaard, K.; Edem, P. E.; Bratteby, K.; Shalgunov, V.; Wilkovitsch, M.; Svatunek, D.; et al. Lipophilicity and Click Reactivity Determine the Performance of Bioorthogonal Tetrazine Tools in Pretargeted In Vivo Chemistry. ACS Pharmacol. Transl. Sci. 2021, 4 (2), 824-833.). Second, tetrazine-decorated resins with longer linkers between the tetrazine moiety and the solid support (those obtained from tetrazines 2, 4 and 6) demonstrated lower trapping efficiencies of TCO-KS254 polymer than resins with shorter or no linker (obtained from tetrazines 1, 3 and 5). This is counterintuitive, since a longer linker should impart greater accessibility of the tetrazine moiety from the liquid phase, which should lead to more efficient, rather than less efficient, IEDDA-mediated trapping. Trapping efficiency for prepared tetrazine-modified resins was preserved for at least 9 days after preparation, which suggests that the resins can have a long shelf-life, which is valuable for routine application.

**Example 7: Extracorporeal clearing of TCO-CC49 from the bloodstream of living rats**

Tetrazine-agarose resin was prepared from tetrazine 2 as described in Example 4, with the following modification: NHS-activated agarose slurry (Pierce/Thermo Fisher Scientific) was used instead of dry agarose, and prepared for coupling as described in the manufacturer's instructions. Control resin (tetrazine-free quenched agarose) was prepared in the same way, but pure PBS without amino-tetrazines was used for the initial incubation. Prepared tetrazine-decorated and tetrazine-free resins were transferred to filtration columns (Biotage) with only the bottom frits present and rinsed with 3-4 column volumes of sterile physiological saline. The total resin bed volume in each column was 1 mL. All operations were performed in a laminar air flow bench to minimize the risk of bacterial contamination. Columns and frits were sterilized in the autoclave before use.

Each column was attached to the extracorporeal clearing circuit consisting of polypropylene and Tygon tubings, Luer adapters and a T-junction with a hydrophobic 0.22 μm PTFE filter acting as a bubble trap (Figure 10). All circuit parts were sterilized by autoclaving before use, and the whole system was flushed by heparinized saline (20 IU/mL heparin in 9 mg/mL NaCl). The total volume of the circuit was about 2 mL.

Radiolabeled TCO-CC49 for the clearing experiment was prepared by incubating TCO-CC49 (0.7 mg in 1.5 mL PBS, 33 nmol TCO) with [$^{111}$In]**8** (15 MBq, 2 ug, 1.5 nmol, 0.05 eq) for 15 min at room temperature. Attachment of $^{111}$In radioactivity to CC49 antibodies was analyzed by radio-HPLC on a Aeris Widepore 3.6 μm C4 column (150 × 4.6 mm) using a gradient of acetonitrile ($CH_3CN$) in water with 0.1% TFA. Gradient conditions: 0-1 min - 5% $CH_3CN$, 1-8 min - linear increase of $CH_3CN$ content to 75%, 8-9 min - 75% $CH_3CN$, 9-9.5 min - linear decrease of $CH_3CN$ content to 5%, 9.5-10 min - 5% $CH_3CN$, elution speed 1.5 mL/min. [$^{111}$In]TCO-CC49 was afforded in a radiochemical yield (RCY) of 95%. Example of HPLC radio-chromatogram of [$^{111}$In]TCO-CC49 is shown in Figure 11.

Female Long-Evans rats (300-350 g body weight) were injected intravenously with [$^{111}$In]TCO-CC49 (150 ug, 4 MBq). After 2 hours, the rats were anesthetized with isoflurane and connected to the clearing circuits: inlets and outlets of

the clearing circuits were connected to catheters inserted into, respectively, tail artery and lateral tail vein of the rats. Blood was pumped through the circuit at a rate of 0.5 mL/min for 60 min. Thus, the total blood volume passed through the circuit equaled 30 mL, which is equivalent to a single passage of all blood through the trapping column (assuming 8% of the rats' weight accounts for blood). Samples of arterial blood were taken for gamma counting before and right after the clearing procedure. Furthermore, samples of blood exiting the column at the end of the clearing procedure were taken for gamma counting to compare untrapped $^{111}$In activity concentrations for tetrazine-decorated and tetrazine-free columns. At the end of the clearing procedure, the catheters were disconnected and the rats were allowed to recover from anesthesia. 22 hours later, the rats were sacrificed and dissected. Samples of blood and internal organs were taken for gamma counting. The trapping columns were rinsed with saline and adsorbed $^{111}$In activity was also measured by gamma counting. $^{111}$In activity concentrations were expressed as % injected dose per gram tissue (%ID/g). In total, 3 rats were used for the experiment, of which 1 rat was connected to a tetrazine-decorated column and 2 rats were connected to tetrazine-free (sham) columns. Measured concentrations of $^{111}$In activity in the blood and organs of rats are shown in Table 3, Table 4 and Figure 12.

Table 3. Concentration of $^{111}$In activity (%ID/g) in the blood of rats before and after extracorporeal clearing procedure

| | Sham columns (n=2) | Tetrazine-decorated column (n=1) |
|---|---|---|
| Arterial blood before clearing | 2.30±0.07 | 2.01 |
| Arterial blood after clearing | 2.20±0.22 | 0.82 |
| Blood exiting the column at the end of clearing | 2.24±0.13 | 0.14 |
| Blood 22 h after clearing | 1.14±0.28 | 0.53 |

Table 4. Concentration of $^{111}$In activity (%ID/g) in the blood and organs of rats at 22 hours after extracorporeal clearing procedure

| | Sham columns (n=2) | Tetrazine-decorated column (n=1) |
|---|---|---|
| Blood | 1.12±0.28 | 0.52 |
| Heart | 0.25±0.06 | 0.11 |
| Lung | 0.61±0.16 | 0.35 |
| Liver | 1.47±0.16 | 1.16 |
| Spleen | 0.60±0.01 | 0.34 |
| Kidney | 0.48±0.05 | 0.26 |
| Muscle | 0.05±0.02 | 0.02 |

As evident from the data in Table 3 and Figure 12, before the extracorporeal clearing procedure, $^{111}$Inactivity concentrations in the whole blood differed only by 13% in the rat connected to the tetrazine-decorated column compared to the rats connected to sham columns (control rats). After the clearing, whole blood activity concentration decreased by 59% in the rat connected to the tetrazine-decorated column, but only by 5% in the control rats. Activity concentration in the blood exiting the column at the end of the extracorporeal clearing was 94% lower in the tetrazine column connected rat than in the control rats, demonstrating efficient trapping of TCO-CC49 from the blood in vivo. At 22 hours after the extracorporeal clearing procedure, the whole blood activity concentration in the rat connected to the tetrazine-decorated column was 53% lower than in the control rats, while activity concentration in kidney, liver, lungs, spleen, heart and muscles was 21-56% lower than in control rats (Table 4). The percentage of the total injected $^{111}$In activity adsorbed on the trapping column was 1.48±0.54% for the control column and 10.2% (6-fold more) for the tetrazine-decorated column.

Existing publications on the trapping of biotin-modified antibodies from the bloodstream of rats with the aid of streptavidin-decorated resin report trapping efficiency of approximately 80% after triple passage through the column (Garkavij, M.; Tennvall, J.; Strand, S. E.; Sjögren, H. O.; JianQing, C.; Nilsson, R.; Isaksson, M. Extracorporeal Whole-Blood Immunoadsorption Enhances Radioimmunotargeting of Iodine-125-Labeled BR96-Biotin Monoclonal Antibody. J. Nucl. Med. 1997, 38 (6), 895-901). Assuming equal trapping efficiency at each passage, this is equivalent to single passage trapping efficiency of 58%. Thus, the trapping efficiency of our IEDDA-based traps is equal to the trapping efficiency of biotin-streptavidin based traps, even though the kinetics of the IEDDA reaction is known to be several orders of magnitude slower than biotin-streptavidin interaction (Stéen, E. J. L.; Edem, P. E.; Nørregaard, K.; Jørgensen, J. T.; Shalgunov, V.; Kjaer, A.; Herth, M. M. Pretargeting in Nuclear Imaging and Radionuclide Therapy: Improving Efficacy of Theranostics and Nanomedicines. Biomaterials 2018, 179, 209-245.). This is a surprising finding, which highlights the

robustness of IEDDA-based trapping.

Remarkably, the extracorporeal trap presented here does not require separation of blood cells from plasma for successful trapping of components dissolved in plasma. This simplifies the construction of the trapping circuits based on the IEDDA principle.

## Claims

1. An extracorporeal clearing trap column comprising a biocompatible solid support to which a chemical entity is attached, **characterized in that** the chemical entity possesses inverse electron demand Diels-Alder cycloaddition reactivity.

2. A clearing trap according to claim 1 wherein the chemical entity of the targeting vector is a diene and the chemical entity in the column is a dienophile or wherein the chemical entity of the targeting vector is a diene and the chemical entity in the column is a dienophile.

3. A clearing trap according to claim 1 or 2 wherein said diene is selected from the group comprising:

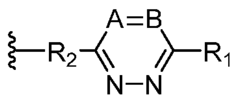

wherein:

A and B are independently N or CH;
$R_1$ is H or a $C_1$-$C_{10}$ alkyl or is selected from the group consisting of:

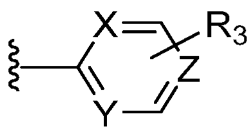

wherein X, Y and Z are independently N or CH;
$R_3$ is a $C_1$-$C_{10}$ alkyl, fluorine, bromine, iodine, carboxylic acid, -CO-$NH_2$, -NH-CO-H, -NH-CO-$CH_3$, or - O-$R_4$, wherein $R_4$ is H, or a $C_1$-$C_{10}$ alkyl or -[-$CH_2$-$CH_2$-O-]$_n$-$CH_3$ wherein n is an integer selected from the group consisting of 1, 2, 3, 4, 5, 6, 7, 8, 9 and 10;
$R_2$ is a $C_1$-$C_{10}$ alkyl, -[-$CH_2$-$CH_2$-O-]$_m$-, wherein in m is an integer selected from the group consisting of 1, 2, 3, 4, 5, 6, 7, 8, 9 and 10, or is selected from the group consisting of:

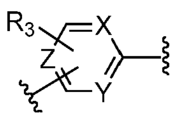

wherein X, Y and Z are independently N or CH;
$R_3$ is a $C_1$-$C_{10}$ alkyl, fluorine, bromine, iodine, carboxylic acid, -CO-$NH_2$, -NH-CO-H, -NH-CO-$CH_3$, or - O-$R_4$, wherein $R_4$ is H, or a $C_1$-$C_{10}$ alkyl or -[-$CH_2$-$CH_2$-O-]$_n$-$CH_3$, wherein n is an integer selected from the group consisting of 1, 2, 3, 4, 5, 6, 7, 8, 9 and 10;
and wherein the complementary trans-cyclooctene derivative is selected from the group comprising:

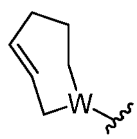

wherein W is -$CH_2$-CH(-O-)-, -$CH_2$-CH(-NH-)-, -C(=O)-N(-)-, -N(-)-C(=O)-, a fused 3-membered ring selected from the group consisting of:

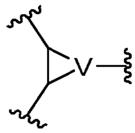

wherein V is N or CH,
a fused 4-membered ring selected from the group consisting of:

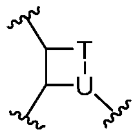

wherein T is O, NH, $CH_2$ or C=O and
U is -CH- or -N-,
or a fused 5-membered ring selected from the group consisting of:

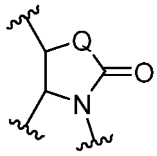 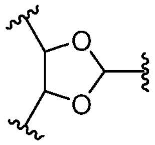 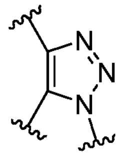

wherein Q is O, S or NH.

4. A clearing trap according to any of the previous claims wherein said diene is a 1,2,4,5-tetrazine derivative selected from the group comprising:

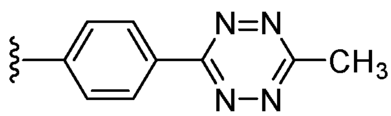
,

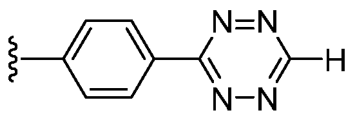

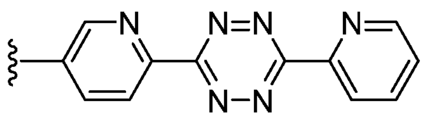

and wherein the complementary dienophile is a trans-cyclooctene derivative selected from the group comprising:

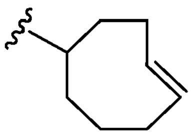 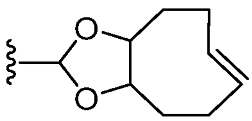 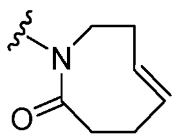 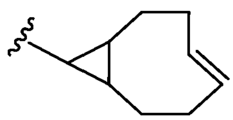

5. A clearing trap according to any of the previous claims wherein said biocompatible solid support is agarose.

6. A clearing trap according to any of the previous claims wherein the chemical entity possessing inverse electron demand Diels-Alder cycloaddition reactivity is attached to the biocompatible solid support by a linker.

7. A clearing trap according to claim 6 wherein the linker is 1 to 100 atoms long and comprises one or more of the following: an amide bond, a urea bond, an alkane chain, a polypeptide, a polypeptoid polymer, polyethylene glycol,

N-(2-hydroxypropyl)methacrylamide, polysarcosine, a thiosuccinimide ring or a triazole ring as structural elements connecting the chemical entity possessing inverse electron-demand Diels-Alder reactivity to the biocompatible solid support.

**8.** A chemical entity with inverse electron demand Diels-Alder cycloaddition reactivity attached to an extracorporeal clearing trap column according to any of claims 1 to 7 for use in the extracorporeal treatment of a disease wherein a targeting vector, comprising a therapeutic or diagnostic agent or a part of such agent and a complementary to the chemical entity in the clearing trap, has been administered to a subject.

**Patentansprüche**

**1.** Extrakorporale Reinigungskolonne, die einen biokompatiblen festen Träger umfasst, an den eine chemische Einheit gebunden ist, **dadurch gekennzeichnet, dass** die chemische Einheit eine inverse elektronenbedürftige Diels-Alder-Cycloadditionsreaktivität aufweist.

**2.** Eine Clearing-Falle gemäß Anspruch 1, wobei die chemische Einheit des Zielvektors ein Dien ist und die chemische Einheit in der Säule ein Dienophil ist, oder wobei die chemische Einheit des Zielvektors ein Dien ist und die chemische Einheit in der Säule ein Dienophil ist.

**3.** Eine Reinigungsfalle gemäß Anspruch 1 oder 2, wobei das Dien aus der Gruppe ausgewählt ist, die umfasst:

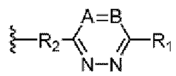

wobei:

A und B unabhängig voneinander N oder CH sind;
$R_1$ H oder ein $C_1$-$C_{10}$ -Alkyl ist oder aus der Gruppe ausgewählt ist, die besteht aus:

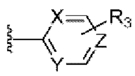

wobei X, Y und Z unabhängig voneinander N oder CH sind;
$R_3$ ein $C_1$-$C_{10}$ -Alkyl, Fluor, Brom, Iod, Carbonsäure, -CO-$NH_2$, - NH-CO-H, -NH-CO-$CH_3$ oder -O-$R_4$, wobei $R_4$ H oder ein $C_1$-$C_{10}$ -Alkyl oder -[-$CH_2$-$CH_2$-O-]$_n$-$CH_3$ ist, wobei n eine ganze Zahl ist, ausgewählt aus der Gruppe bestehend aus 1, 2, 3, 4, 5, 6, 7, 8, 9 und 10;
$R_2$ ein $C_1$-$C_{10}$ -Alkylrest, -[-$CH_2$-$CH_2$-O-]$_m$- ist, wobei m eine ganze Zahl aus der Gruppe bestehend aus 1, 2, 3, 4, 5, 6, 7, 8, 9 und 10 ist, oder aus der Gruppe ausgewählt ist, bestehend aus:

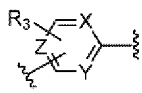

wobei X, Y und Z unabhängig voneinander N oder CH sind;
$R_3$ ein $C_1$-$C_{10}$ -Alkyl, Fluor, Brom, Iod, Carbonsäure, -CO-$NH_2$, - NH-CO-H, -NH-CO-$CH_3$ oder -O-R, wobei $R_4$ H oder ein $C_1$-$C_{10}$ -Alkyl oder -[-$CH_2$-$CH_2$-O-]$_2$-$CH_3$ ist, wobei n eine ganze Zahl ist, ausgewählt aus der Gruppe bestehend aus 1, 2, 3, 4, 5, 6, 7, 8, 9 und 10 ausgewählt ist;
und wobei das komplementäre trans-Cycloocten-Derivat ausgewählt ist aus der Gruppe bestehend aus:

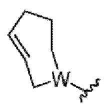

wobei W -$CH_2$-CH(-O-)-, -$CH_2$-CH(-NH-)-, -C(=O)-N(-)-, -N(-)-C(=O)-, einem kondensierten 3-gliedrigen Ring, ausgewählt aus der Gruppe bestehend aus:

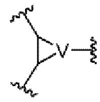

wobei V N oder CH ist,
einem kondensierten 4-gliedrigen Ring, ausgewählt aus der Gruppe bestehend aus:

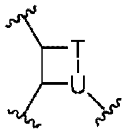

wobei T O, NH, $CH_2$ oder C=O ist und
U -CH- oder -N- ist,
oder einem kondensierten 5-gliedrigen Ring, ausgewählt aus der Gruppe bestehend aus:

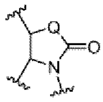 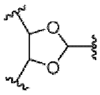 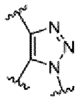

wobei Q O, S oder NH ist.

**4.** Eine Clearing-Falle gemäß einem der vorherigen Ansprüche, wobei das Dien ein 1,2,4,5-Tetrazin-Derivat ist, ausgewählt aus der Gruppe bestehend aus:

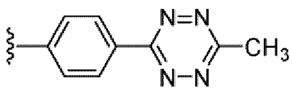,

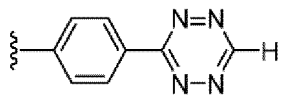

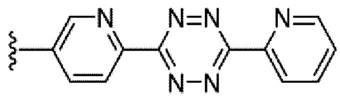

und wobei das komplementäre Dienophil ein trans-Cycloocten-Derivat ist, ausgewählt aus der Gruppe bestehend aus:

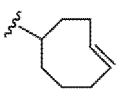 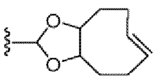 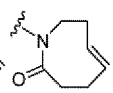 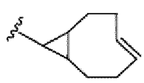

**5.** Eine Clearing-Falle gemäß einem der vorherigen Ansprüche, wobei der biokompatible feste Träger Agarose ist.

**6.** Eine Klärungsfalle gemäß einem der vorherigen Ansprüche, bei der die chemische Einheit, die eine inverse Elektronenbedarfs-Diels-Alder-Cycloadditionsreaktivität aufweist, über einen Linker an den biokompatiblen festen Träger gebunden ist.

**7.** Eine Clearing-Falle gemäß Anspruch 6, wobei der Linker 1 bis 100 Atome lang ist und eines oder mehrere der folgenden Elemente umfasst: eine Amidbindung, eine Harnstoffbindung, eine Alkankette, ein Polypeptid, ein Polypeptoidpolymer, Polyethylenglykol, N-(2-Hydroxypropyl)methacrylamid, Polysarcosin, einen Thiosuccinimidring oder einen Triazolring als Strukturelemente, die die chemische Einheit mit inverser Elektronenbedarfs-Diels-Alder-Reaktivität mit dem biokompatiblen festen Träger verbinden.

8. Chemische Einheit mit inverser Elektronenbedarfs-Diels-Alder-Cycloadditionsreaktivität, die an eine extrakorporale Clearings-Trap-Säule gemäß einem der Ansprüche 1 bis 7 angebracht ist, zur Verwendung bei der extrakorporalen Behandlung einer Erkrankung, wobei einem Patienten ein Zielvektor verabreicht wurde, der ein therapeutisches oder diagnostisches Mittel oder einen Teil eines solchen Mittels und ein Komplementärteil zu der chemischen Einheit in der Clearings-Trap umfasst.

**Revendications**

1. Colonne de piège de dégagement extracorporel comprenant un support solide biocompatible auquel est attachée une entité chimique, **caractérisée en ce que** l'entité chimique possède une réactivité de cycloaddition Diels-Alder à demande inverse d'électron.

2. Piège de dégagement selon la revendication 1, dans lequel l'entité chimique du vecteur de ciblage est un diène et l'entité chimique dans la colonne est un diénophile ou dans lequel l'entité chimique du vecteur de ciblage est un diène et l'entité chimique dans la colonne est un diénophile.

3. Piège de dégagement selon la revendication 1 ou 2, dans lequel ledit diène est choisi dans le groupe constitué de :

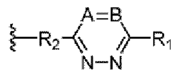

dans lequel :

A et B sont indépendamment N ou CH ;
$R_1$ est H ou alkyle en $C_1$-$C_{10}$ ou est choisi dans le groupe constitué de :

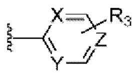

dans lequel, X, Y et Z sont indépendamment N ou CH ;
$R_3$ est alkyle en $C_1$-$C_{10}$, fluor, brome, iode, acide carboxylique, -CO-$NH_2$, -NH-CO-H, -NH-CO-$CH_3$, ou -O-$R_4$, dans lequel $R_4$ est H, ou alkyle en $C_1$-$C_{10}$ ou -[$CH_2$-$CH_2$-O-]$_n$-$CH_3$ dans lequel n est un entier choisi dans le groupe constitué de 1, 2, 3, 4, 5, 6, 7, 8, 9 et 10 ;
$R_2$ est alkyle en $C_1$-$C_{10}$, -[$CH_2$-$CH_2$-O-]$_m$-, dans lequel m est un entier choisi dans le groupe constitué de 1, 2, 3, 4, 5, 6, 7, 8, 9 et 10, ou est choisi dans le groupe constitué de :

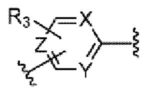

dans lequel, X, Y et Z sont indépendamment N ou CH ;
$R_3$ est alkyle en $C_1$-$C_{10}$, fluor, brome, iode, acide carboxylique, -CO-$NH_2$, -NH-CO-H, -NH-CO-$CH_3$, ou -O-R, dans lequel $R_4$ est H, ou alkyle en $C_1$-$C_{10}$ ou -[$CH_2$-$CH_2$-O-]$_n$-$CH_3$ dans lequel n est un entier choisi dans le groupe constitué de 1, 2, 3, 4, 5, 6, 7, 8, 9 et 10 ;
et dans lequel le dérivé trans-cyclooctène complémentaire est choisi dans le groupe constitué de :

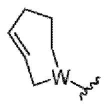

dans lequel W est -$CH_2$-CH(-O-)-, -$CH_2$-CH(-NH-)-, -C(=O)-N(-)-, - N(-)-C(=O)-, un cycle condensé à 3 chaînons choisi dans le groupe constitué de :

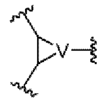

dans lequel V est N ou CH,
un cycle condensé à 4 chaînons choisi dans le groupe constitué de :

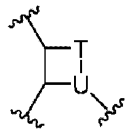

dans lequel T est O, NH, $CH_2$ ou C=O et
U est -CH- ou -N-,
un cycle condensé à 5 chaînons choisi dans le groupe constitué de :

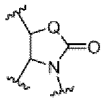 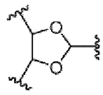 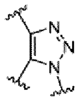

dans lequel Q est O, S ou NH.

**4.** Piège de dégagement selon l'une quelconque des revendications précédentes, dans lequel ledit diène est un dérivé de la 1,2,4,5-tétrazine choisi dans le groupe constitué de :

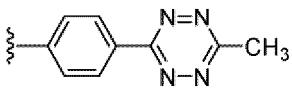 ,

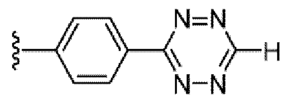

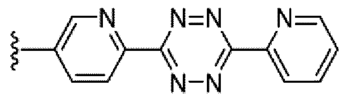

et dans lequel le diénophile complémentaire est un dérivé trans-cyclooctène choisi dans le groupe constitué de :

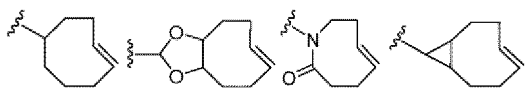

**5.** Piège de dégagement selon l'une quelconque des revendications précédentes, dans lequel ledit support solide biocompatible est de l'agarose.

**6.** Piège de dégagement selon l'une quelconque des revendications précédentes, dans lequel l'entité chimique possédant une réactivité de cycloaddition Diels-Alder à demande inverse d'électron est attachée au support solide biocompatible par un lieur.

**7.** Piège de dégagement selon la revendication 6, dans lequel le lieur a une longueur de 1 à 100 atomes et comprend un ou plusieurs des éléments suivants : une liaison amide, une liaison urée, une chaîne alcane, un polypeptide, un polymère polypeptoïde, du polyéthylène glycol, du N-(2-hydroxypropyl)méthacrylamide, de la polysarcosine, un cycle thiosuccinimide ou un cycle triazole comme éléments structuraux connectant l'entité chimique possédant une réactivité Diels-Alder à demande inverse d'électron au support solide biocompatible.

**8.** Entité chimique avec une réactivité de cycloaddition Diels-Alder à demande inverse d'électron attachée à une colonne de piège de dégagement extracorporel selon l'une quelconque des revendications 1 à 7 pour une utilisation dans le traitement extracorporel d'une maladie dans lequel un vecteur de ciblage, comprenant un agent thérapeutique ou diagnostique ou une partie d'un tel agent et un complémentaire de l'entité chimique dans le piège de dégagement, a été administré à un sujet.

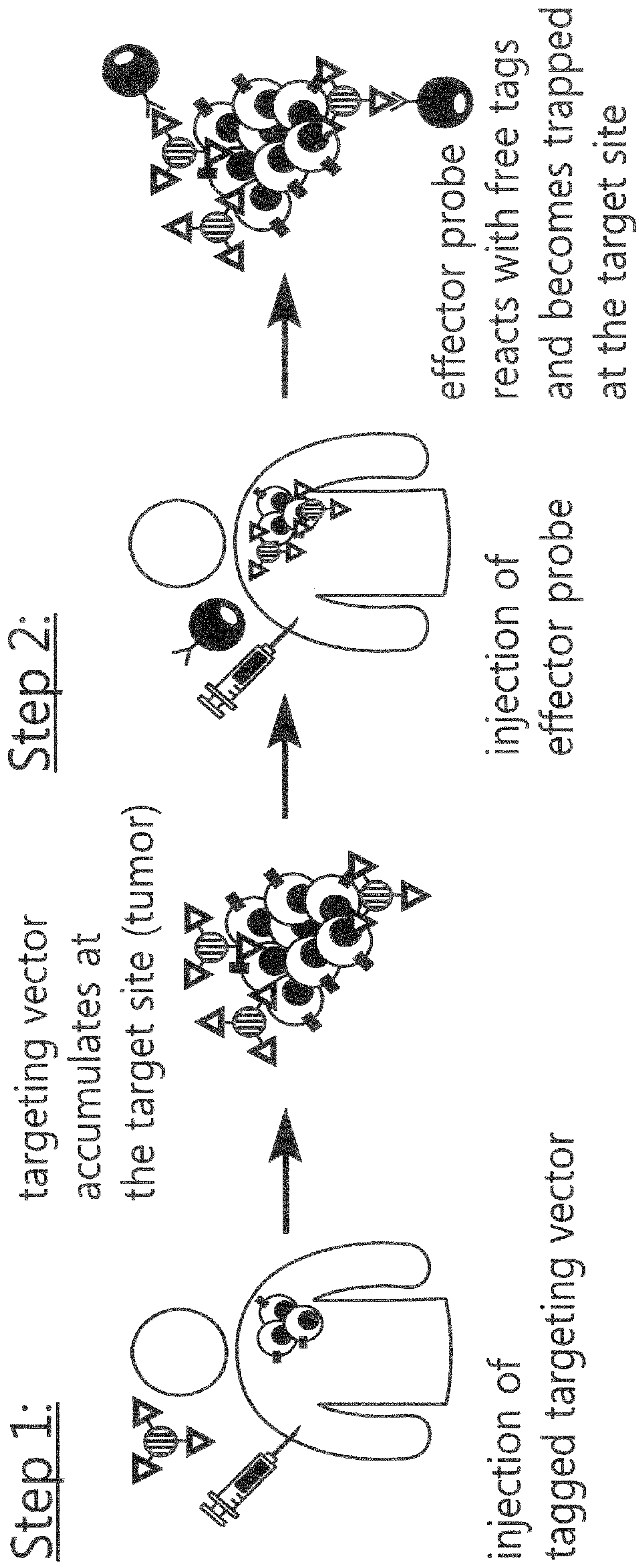
Step 1:
injection of tagged targeting vector
targeting vector accumulates at the target site (tumor)
Step 2:
injection of effector probe
effector probe reacts with free tags and becomes trapped at the target site

FIG. 1

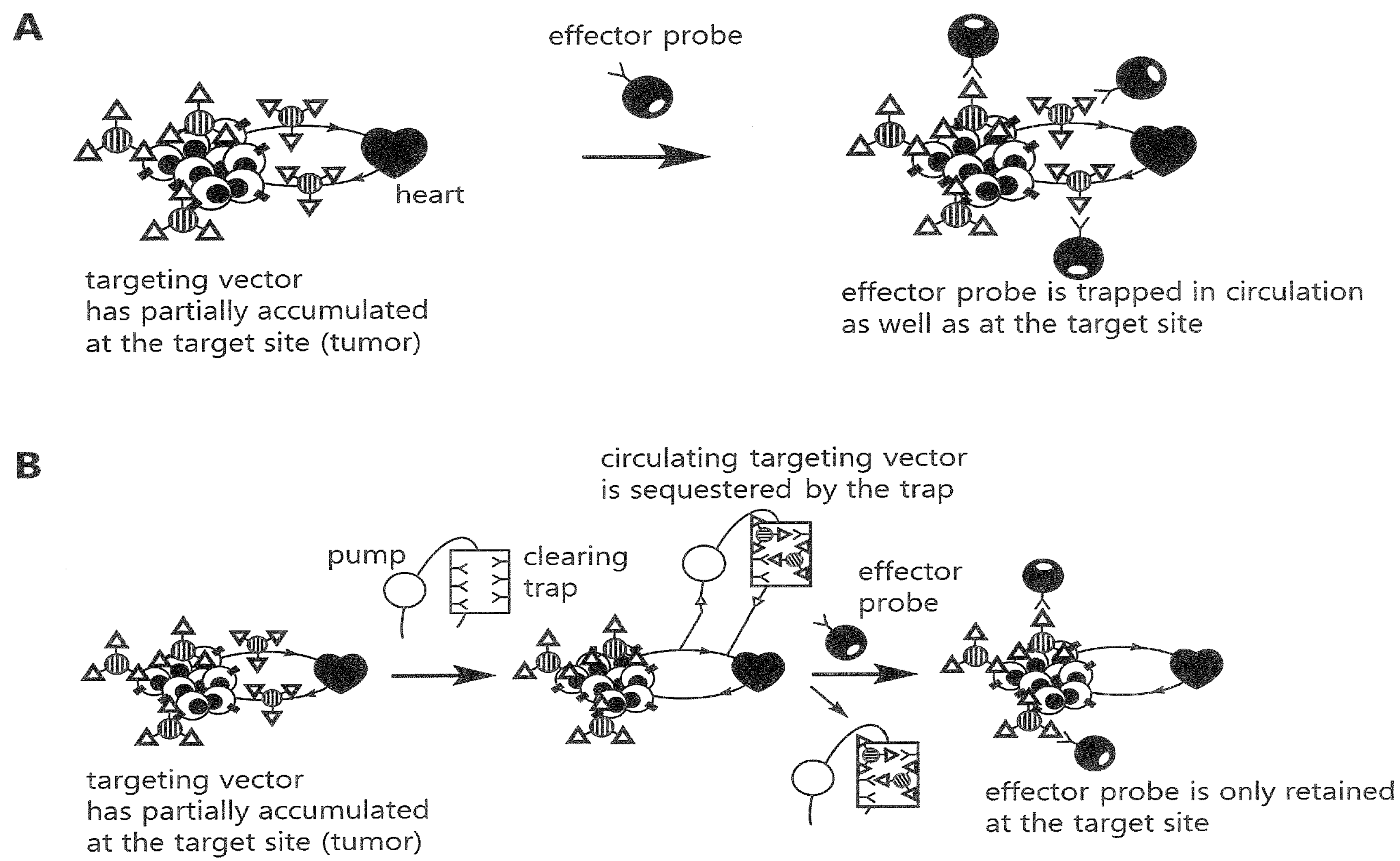
A
effector probe
heart
targeting vector
has partially accumulated
at the target site (tumor)
effector probe is trapped in circulation
as well as at the target site
B
pump
clearing
trap
circulating targeting vector
is sequestered by the trap
effector
probe
targeting vector
has partially accumulated
at the target site (tumor)
effector probe is only retained
at the target site

FIG. 2

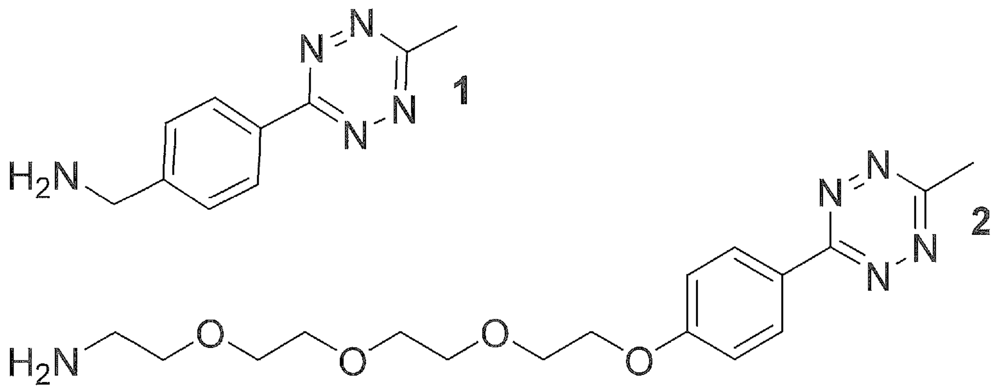

1

2

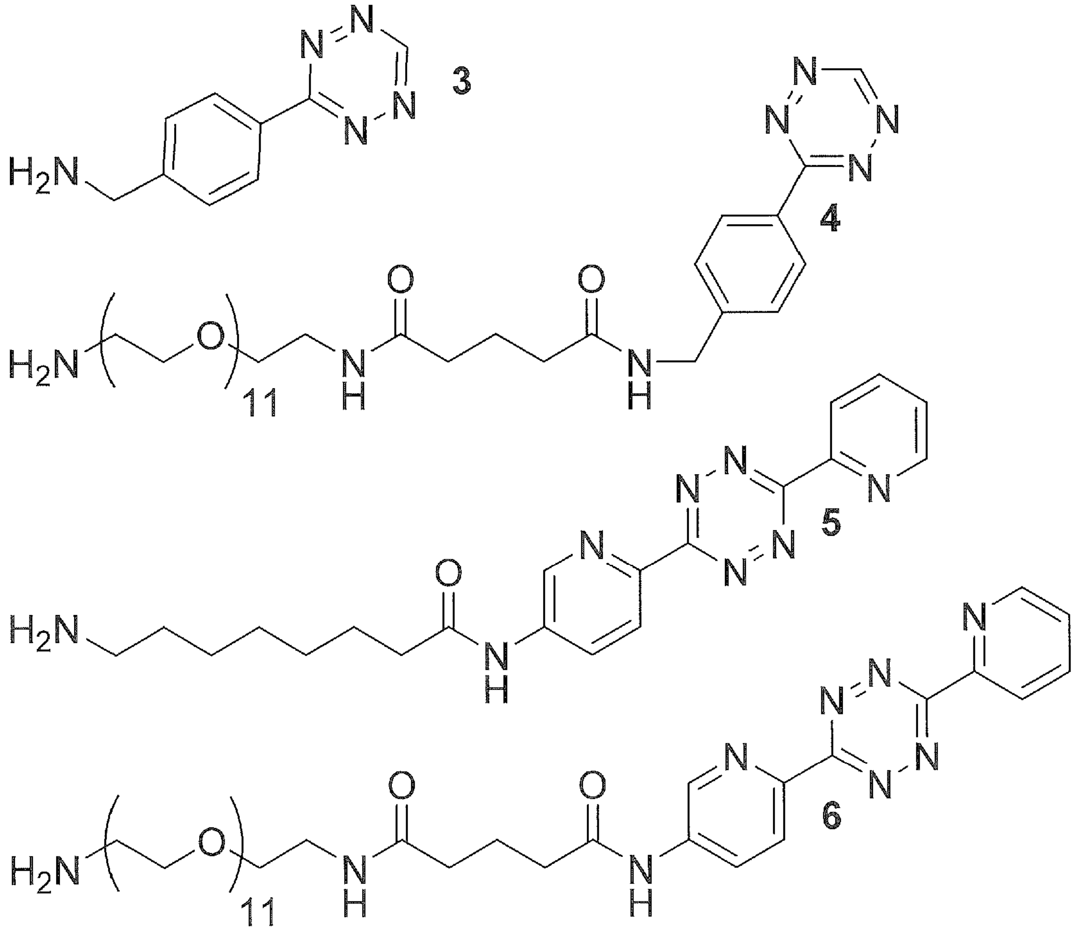

3

4

5

6

FIG. 3

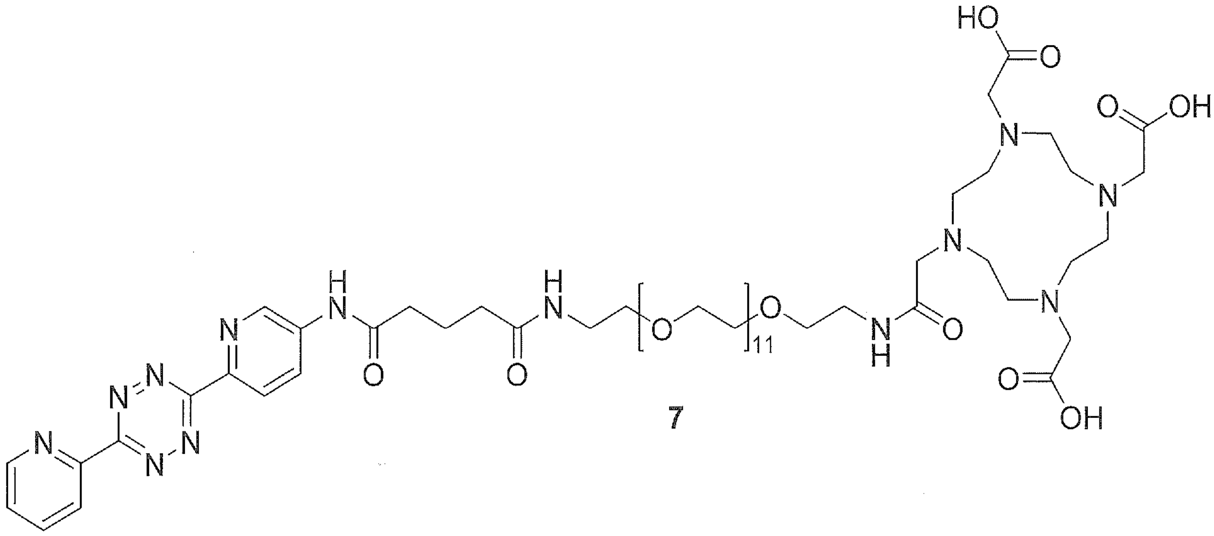

7

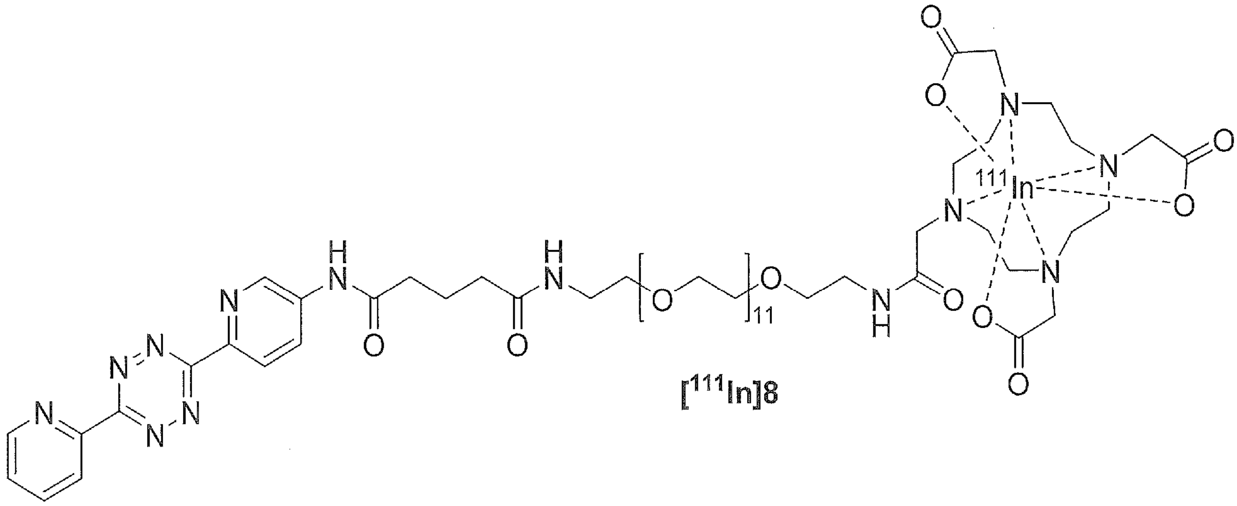

[$^{111}$In]8

FIG. 4

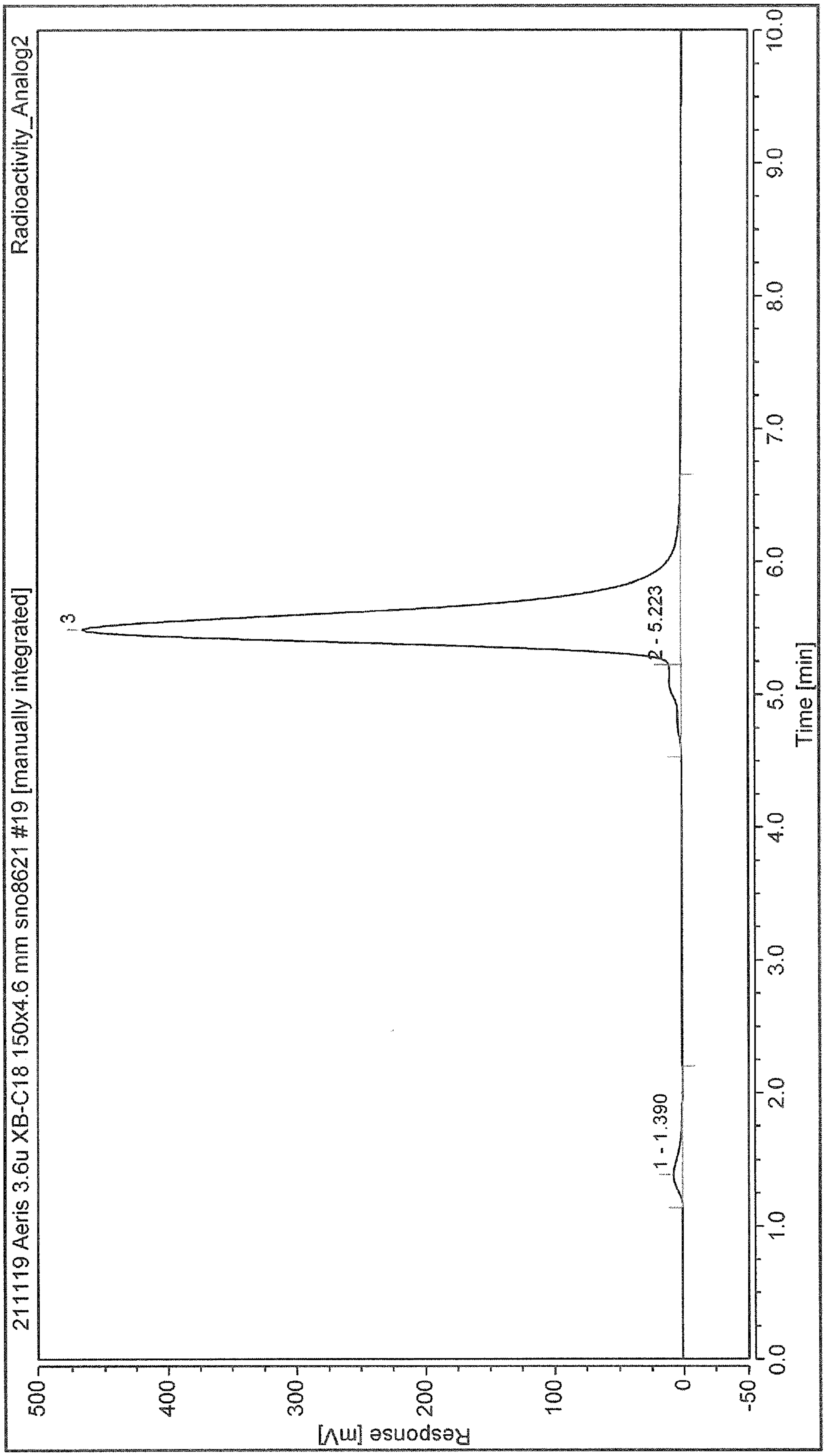
211119 Aeris 3.6u XB-C18 150x4.6 mm sno8621 #19 [manually integrated]
Radioactivity_Analog2
3
2 - 5.223
1 - 1.390
Response [mV]
500
400
300
200
100
0
-50
0.0
1.0
2.0
3.0
4.0
5.0
6.0
7.0
8.0
9.0
10.0
Time [min]

FIG. 5

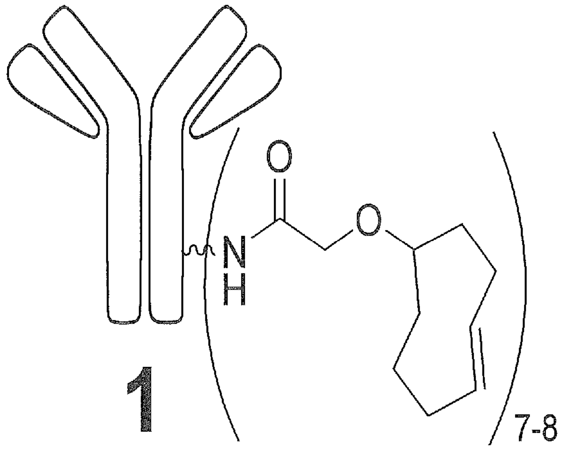

1

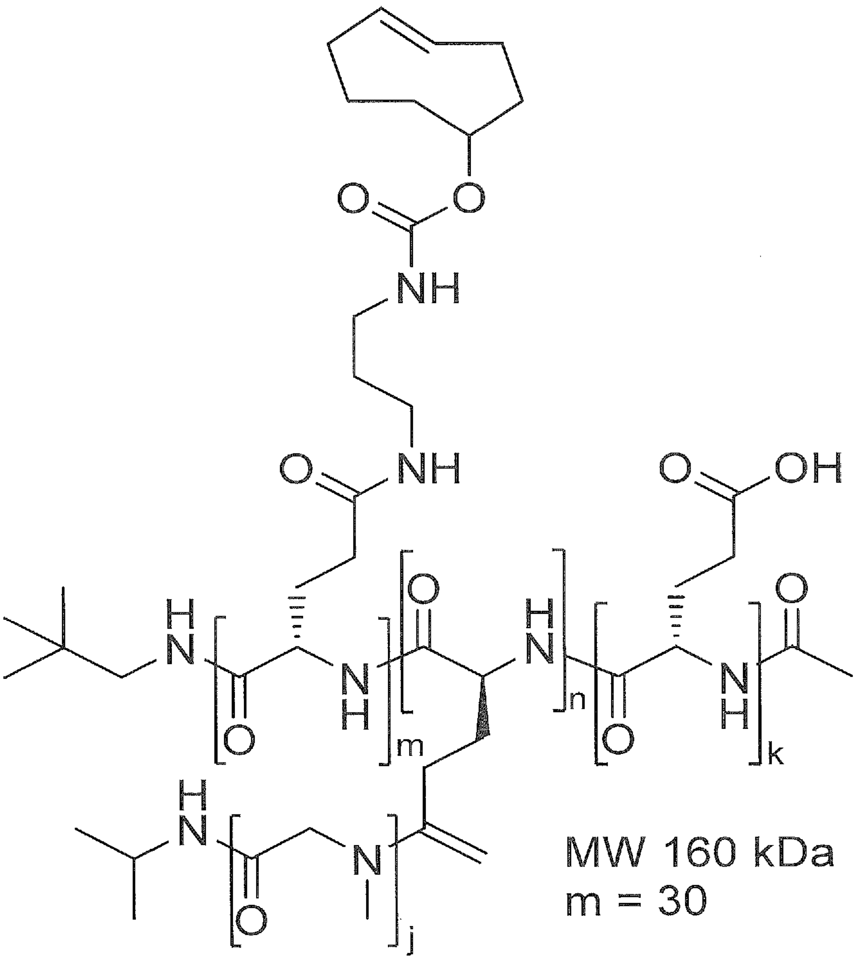

MW 160 kDa
m = 30

2

FIG. 6

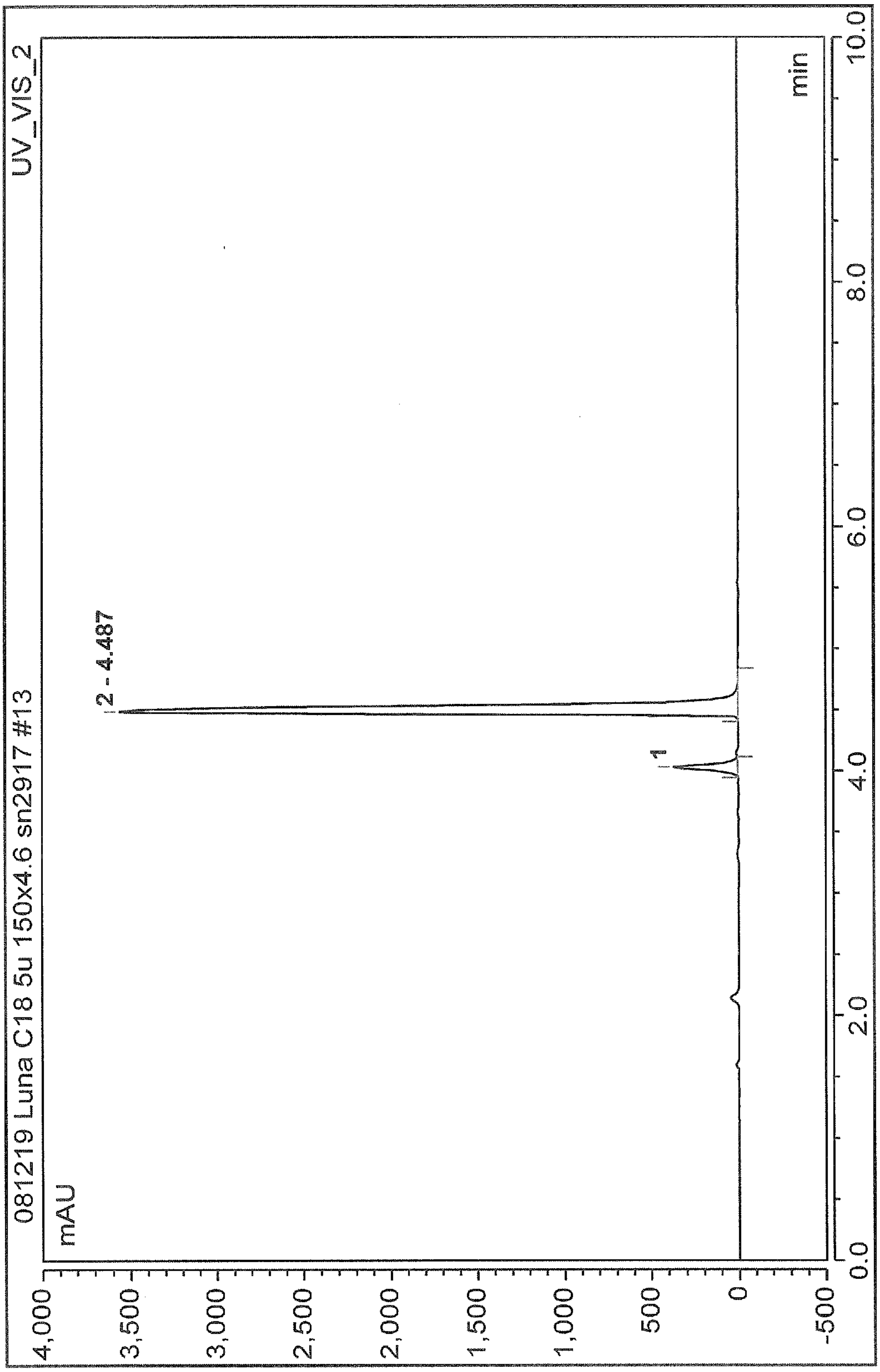


HPLC Chromatogram of Tetrazine 1

FIG. 7A

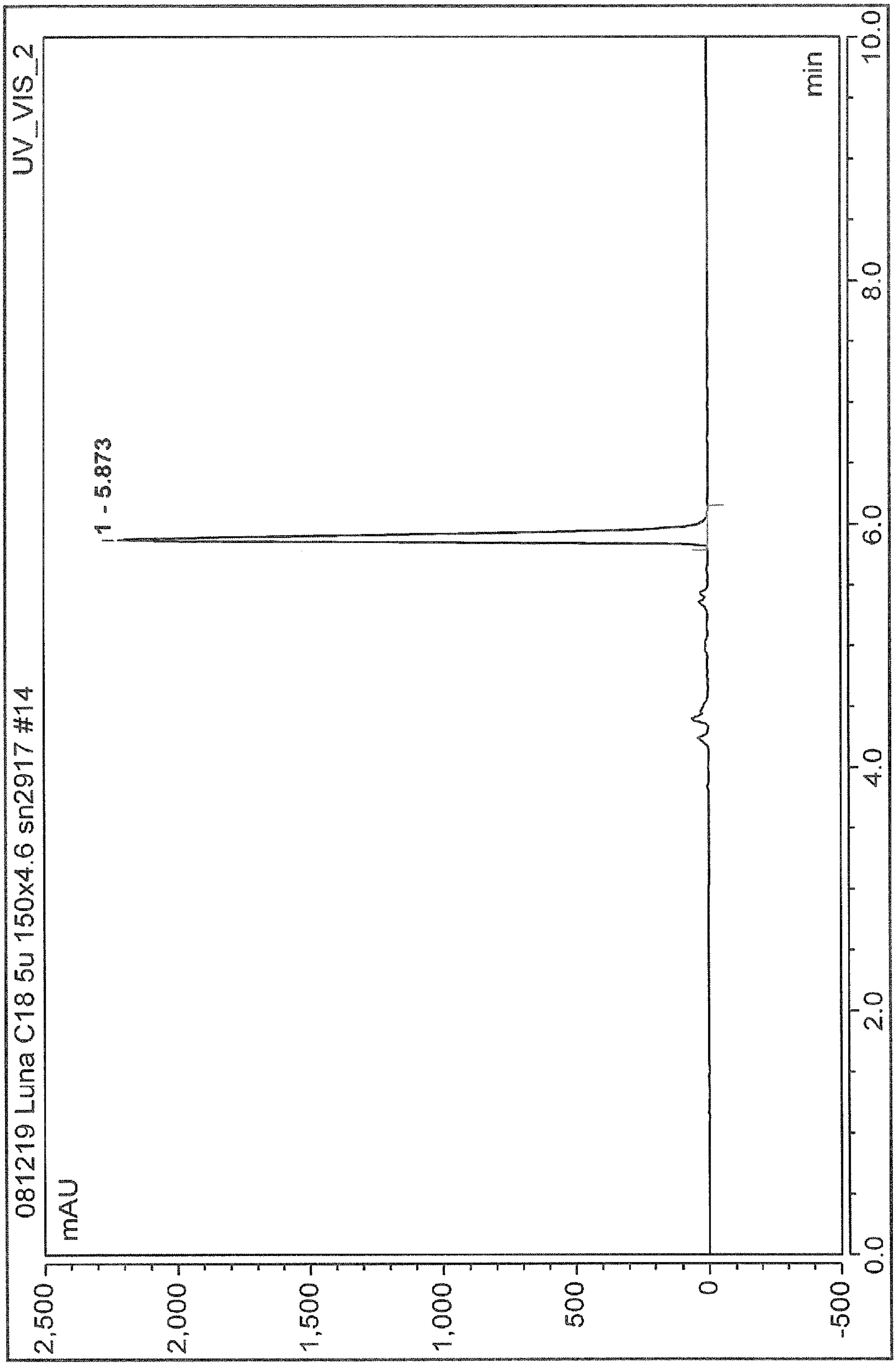


HPLC Chromatogram of Tetrazine 2

FIG. 7B

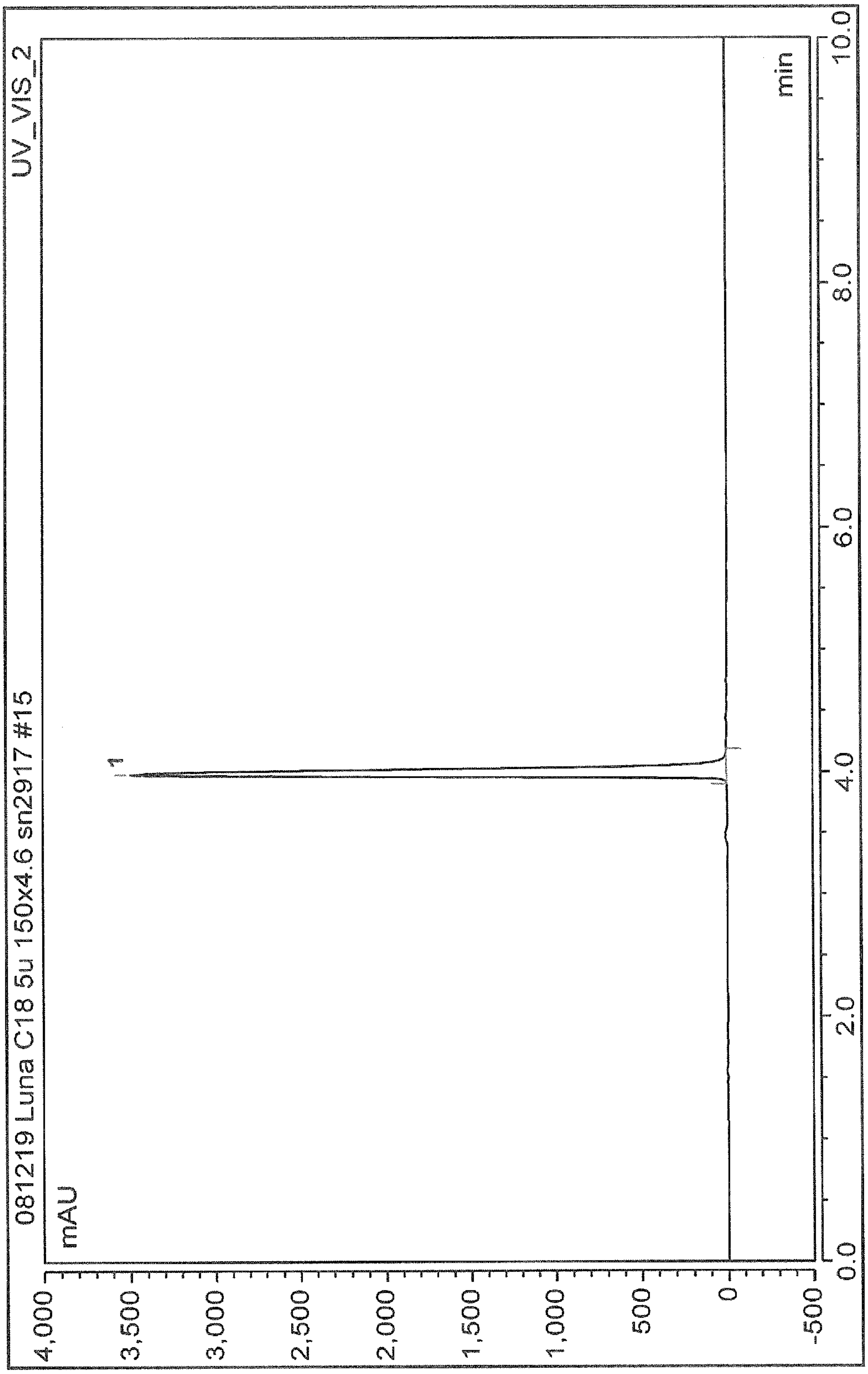


HPLC Chromatogram of Tetrazine 3

FIG. 7C

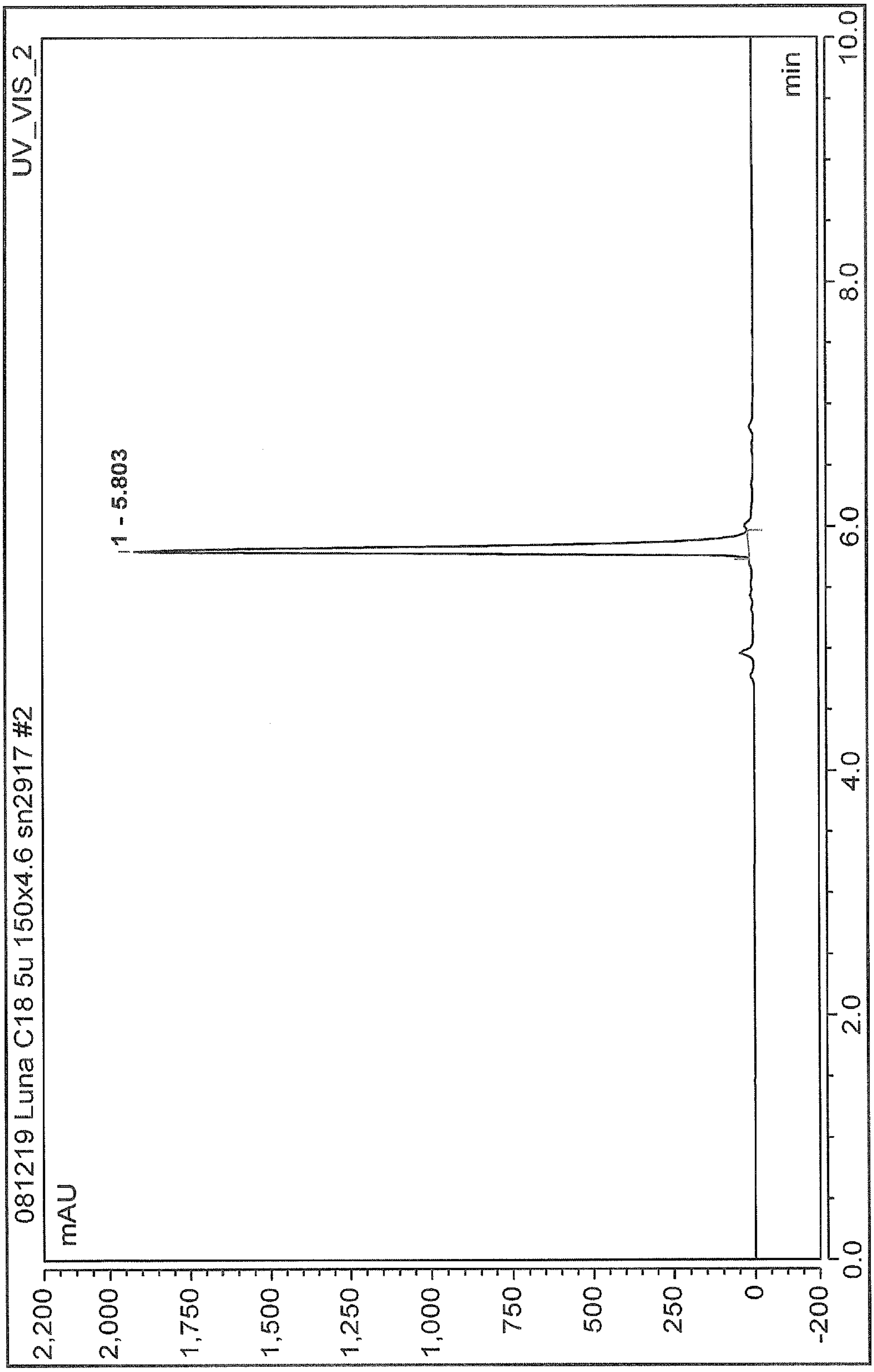


HPLC Chromatogram of Tetrazine 4

FIG. 7D

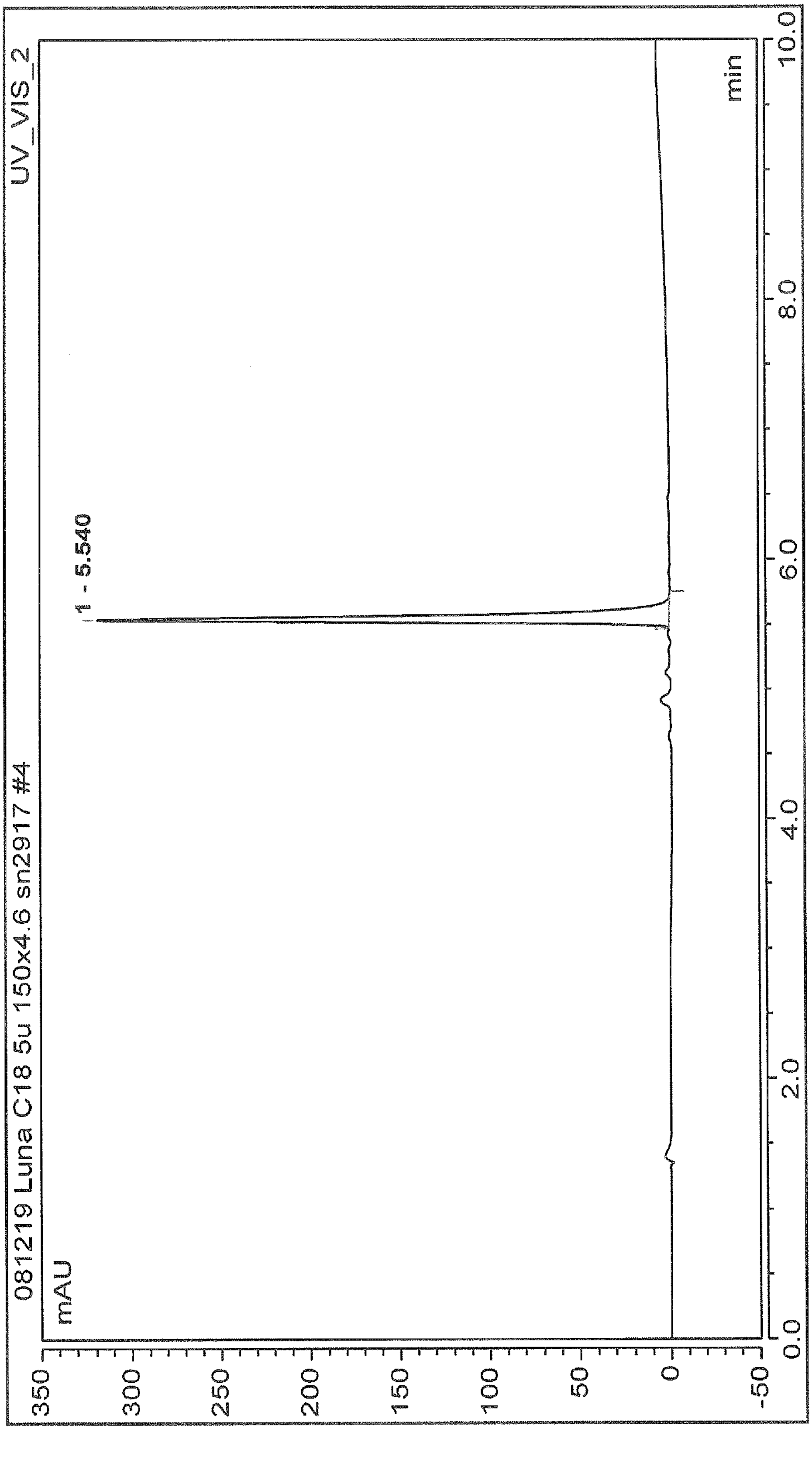


HPLC Chromatogram of Tetrazine 5

FIG. 7E

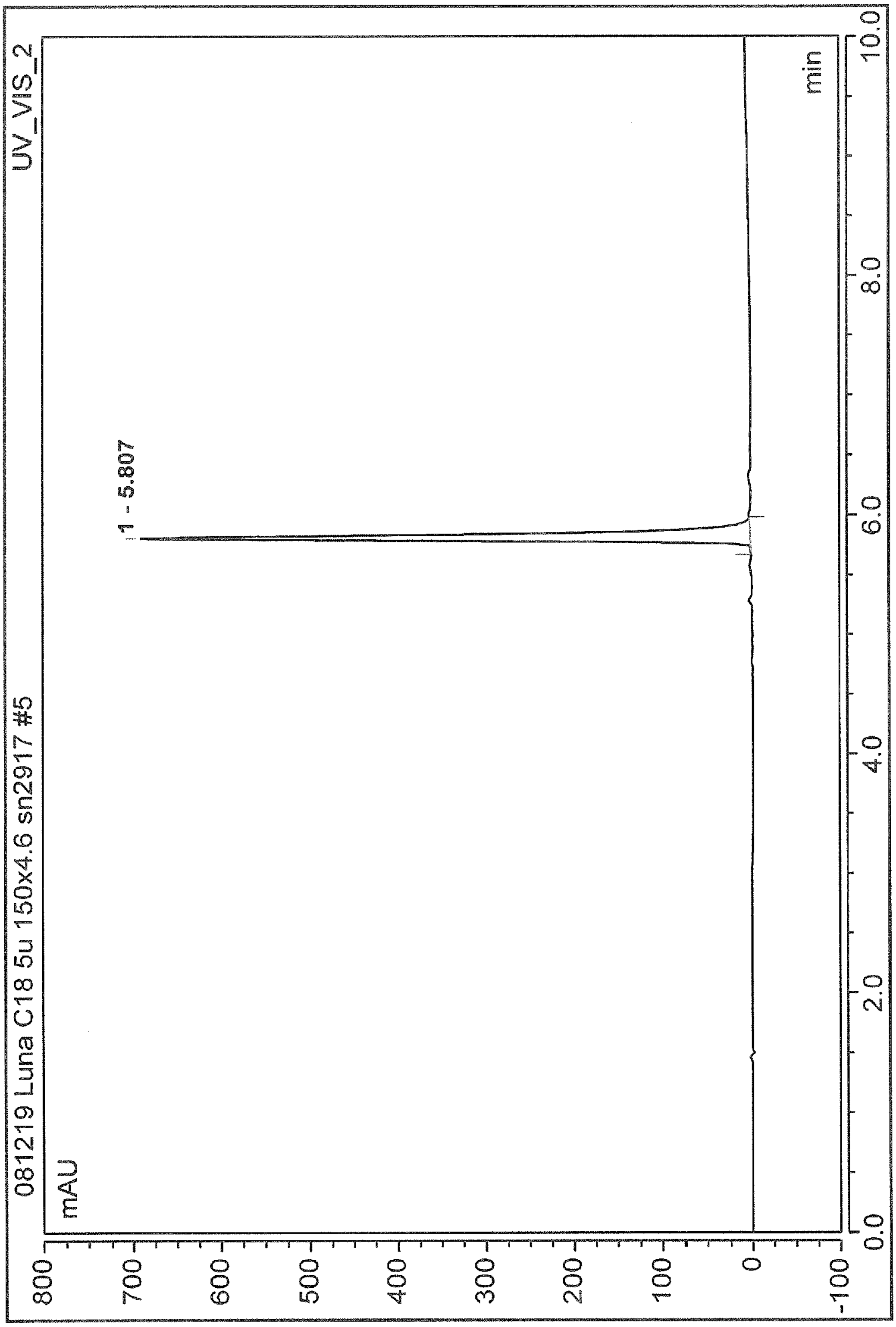


HPLC Chromatogram of Tetrazine 6

FIG. 7F

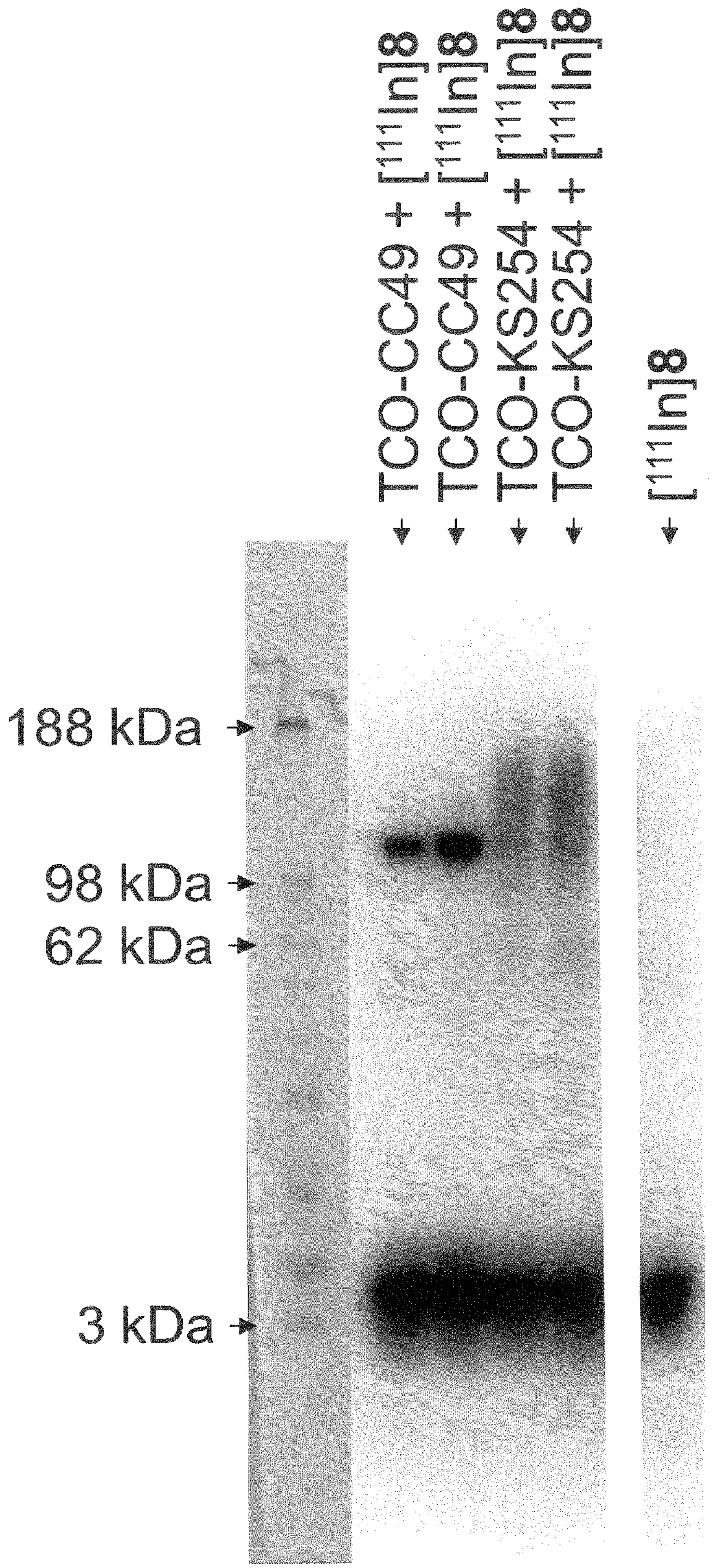
TCO-CC49 + [111In]8
TCO-CC49 + [111In]8
TCO-KS254 + [111In]8
TCO-KS254 + [111In]8
[111In]8
188 kDa
98 kDa
62 kDa
3 kDa

FIG. 8

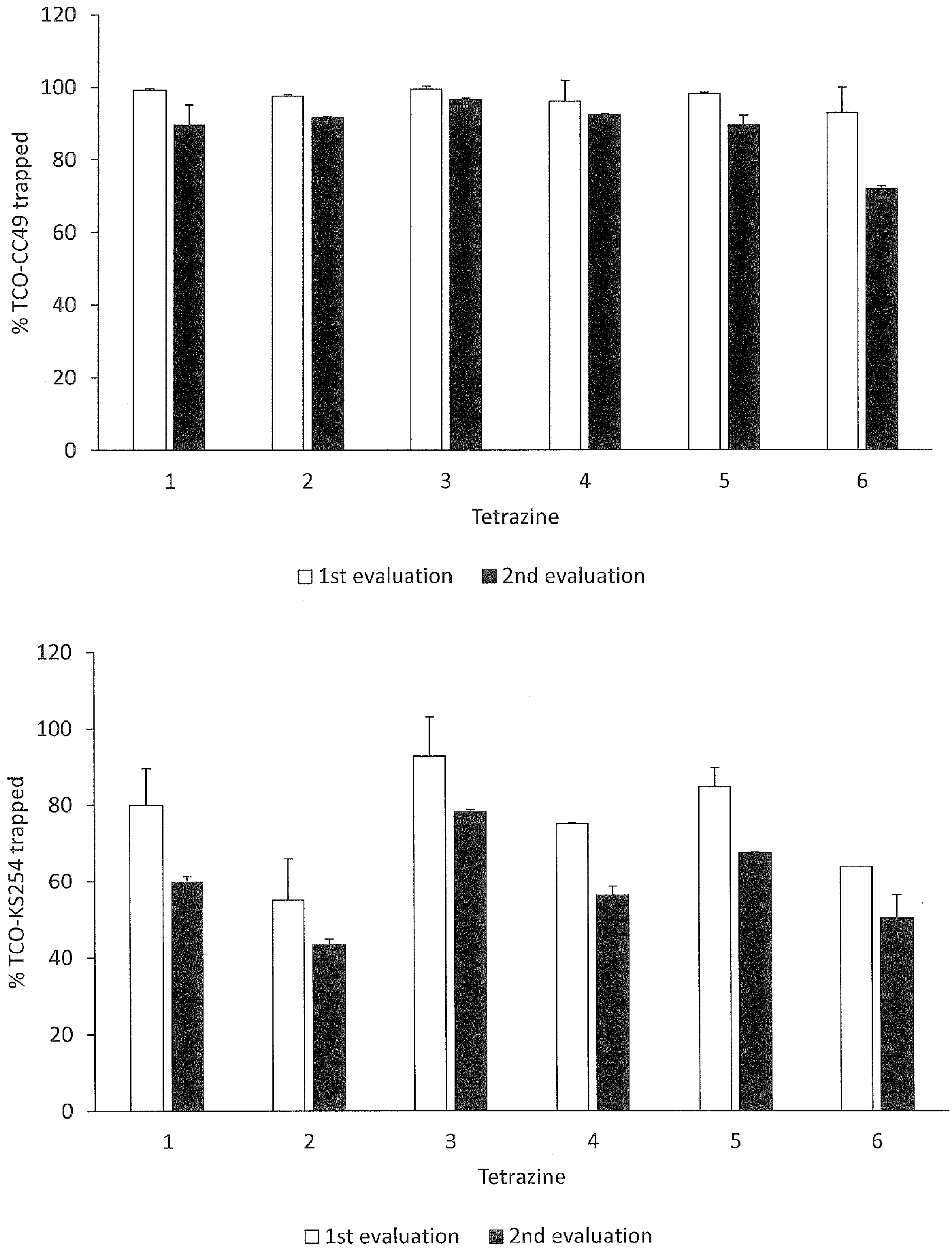
120
100
80
60
40
20
0
% TCO-CC49 trapped
1
2
3
4
5
6
Tetrazine
1st evaluation
2nd evaluation
120
100
80
60
40
20
0
% TCO-KS254 trapped
1
2
3
4
5
6
Tetrazine
1st evaluation
2nd evaluation

FIG. 9

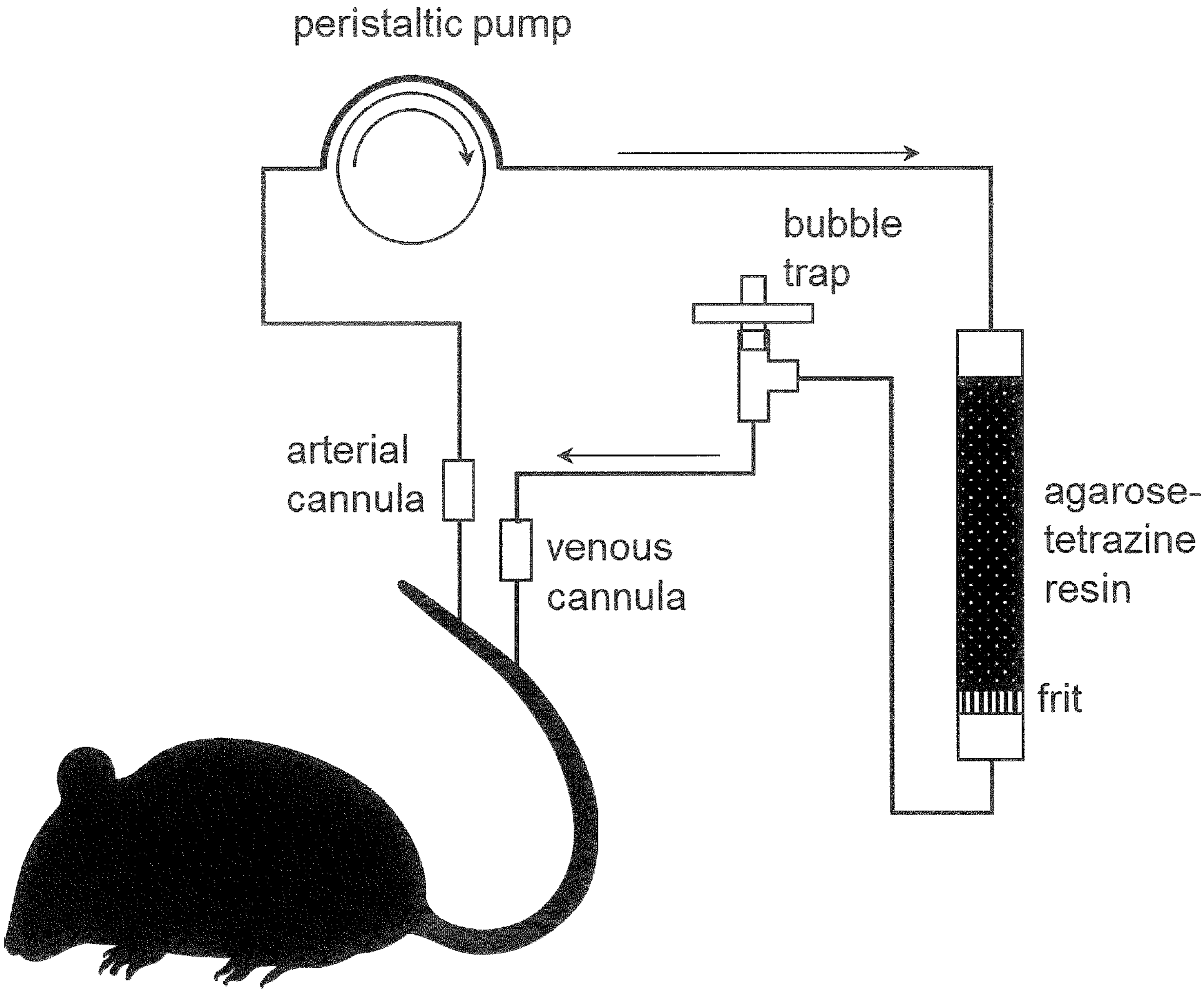
peristaltic pump
bubble
trap
arterial
cannula
venous
cannula
agarose-
tetrazine
resin
frit

FIG. 10

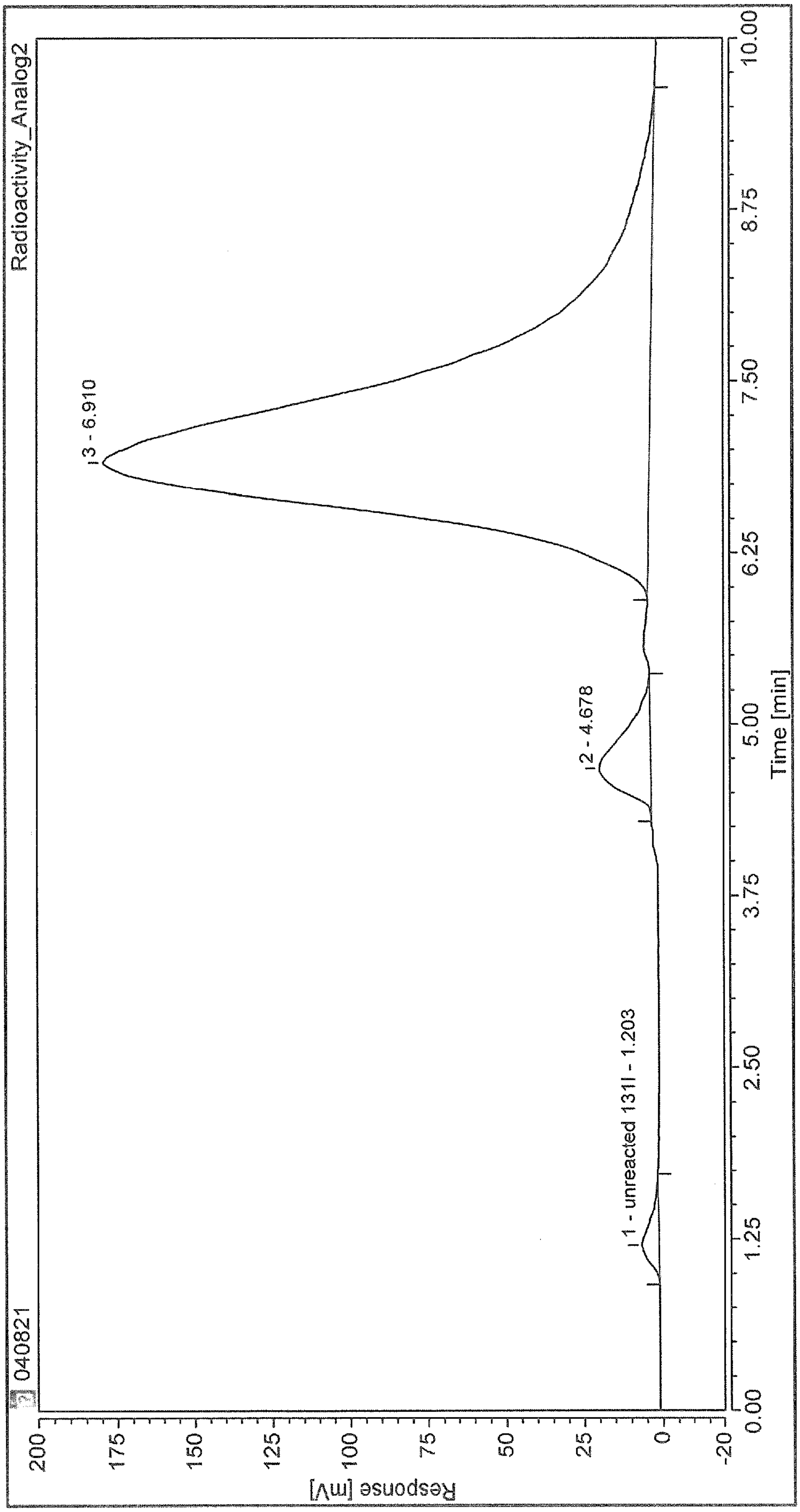
Radioactivity_Analog2
040821
3 - 6.910
2 - 4.678
1 - unreacted 131I - 1.203
Response [mV]
200
175
150
125
100
75
50
25
0
-20
Time [min]
0.00
1.25
2.50
3.75
5.00
6.25
7.50
8.75
10.00

FIG. 11

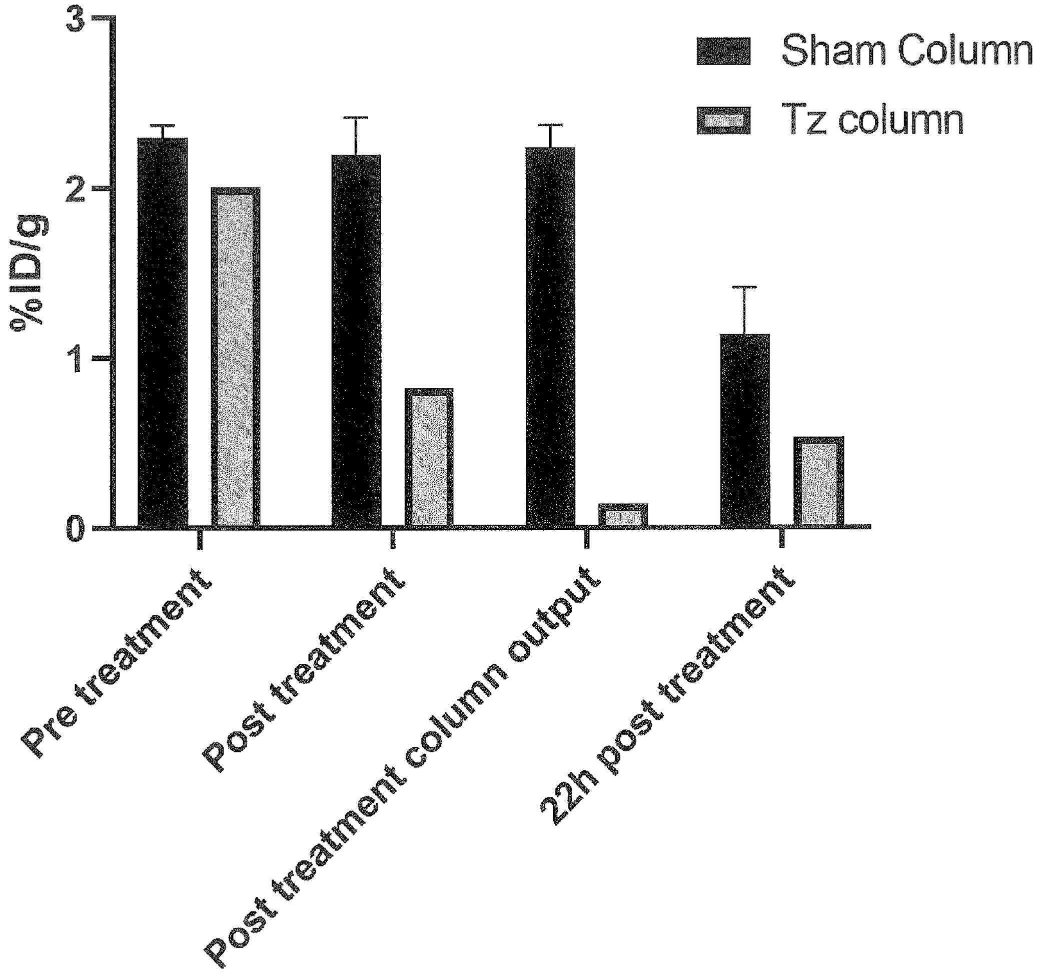

3
2
1
0
%ID/g
Sham Column
Tz column
Pre treatment
Post treatment
Post treatment column output
22h post treatment


FIG. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 9913921 B2
- US 2019247513 A


### Non-patent literature cited in the description


- **OLIVEIRA, B. L.** ; **GUO, Z.** ; **BERNARDES, G. J. L**. Inverse Electron Demand Diels-Alder Reactions in Chemical Biology. *Chem. Soc. Rev.*, 2017, vol. 46 (16), 4895-4950
- **STÉEN, E. J. L.** ; **JØRGENSEN, J. T.** ; **DENK, C.** ; **BATTISTI, U. M.** ; **NØRREGAARD, K.** ; **EDEM, P. E.** ; **BRATTEBY, K.** ; **SHALGUNOV, V.** ; **WILKOVITSCH, M.** ; **SVATUNEK, D. et al.** Lipophilicity and Click Reactivity Determine the Performance of Bioorthogonal Tetrazine Tools in Pretargeted In Vivo Chemistry. *ACS Pharmacol. Transl. Sci.*, 2021, vol. 4 (2), 824-833
- *Polymer Advanced Technology*, 2014, vol. 25, 448-460
- **ALTAI M** ; **MEMBRENO R** ; **COOK B** ; **TOLMACHEV V** ; **ZEGLIS BM**. Pretargeted Imaging and Therapy. *J Nucl Med*, 2017, vol. 58, 1553-9
- **STÉEN EJL** ; **EDEM PE** ; **NØRREGAARD K** ; **JØRGENSEN JT** ; **SHALGUNOV V** ; **KJAER A et al.** Pretargeting in nuclear imaging and radionuclide therapy: Improving efficacy of theranostics and nanomedicines. *Biomaterials*, 2018, vol. 179, 209-45
- **ROSSIN, R.** ; **VERKERK, P. R.** ; **VAN DEN BOSCH, S. M.** ; **VULDERS, R. C. M.** ; **VEREL, I.** ; **LUB, J.** ; **ROBILLARD, M. S.** In Vivo Chemistry for Pretargeted Tumor Imaging in Live Mice. *Angew Chem Int Edit*, 2010, vol. 49 (19), 3375-3378
- **ROSSIN, R.** ; **VAN DUIJNHOVEN, S. M. J.** ; **LÄPPCHEN, T.** ; **VAN DEN BOSCH, S. M.** ; **ROBILLARD, M. S.** Trans-Cyclooctene Tag with Improved Properties for Tumor Pretargeting with the Diels-Alder Reaction. *Mol. Pharm.*, 2014, vol. 11 (9), 3090-3096
- **ROSSIN, R.** ; **VAN DEN BOSCH, S. M.** ; **TEN HOEVE, W.** ; **CARVELLI, M.** ; **VERSTEEGEN, R. M.** ; **LUB, J.** ; **ROBILLARD, M. S**. Highly Reactive Trans -Cyclooctene Tags with Improved Stability for Diels-Alder Chemistry in Living Systems. *Bioconjug. Chem.*, 2013, vol. 24 (7), 1210-1217
- **STÉEN, E. J. L.** ; **JØRGENSEN, J. T.** ; **JOHANN, K.** ; **NØRREGAARD, K.** ; **SOHR, B.** ; **SVATUNEK, D.** ; **BIRKE, A.** ; **SHALGUNOV, V.** ; **EDEM, P. E.** ; **ROSSIN, R. et al.** Trans -Cyclooctene-Functionalized PeptoBrushes with Improved Reaction Kinetics of the Tetrazine Ligation for Pretargeted Nuclear Imaging. *ACS Nano*, 2020, vol. 14 (1), 568-584
- **GARKAVIJ, M.** ; **TENNVALL, J.** ; **STRAND, S. E.** ; **SJÖGREN, H. O.** ; **JIANQING, C.** ; **NILSSON, R.** ; **ISAKSSON, M**. Extracorporeal Whole-Blood Immunoadsorption Enhances Radioimmunotargeting of Iodine-125-Labeled BR96-Biotin Monoclonal Antibody. *J. Nucl. Med.*, 1997, vol. 38 (6), 895-901
- **STÉEN, E. J. L.** ; **EDEM, P. E.** ; **NØRREGAARD, K.** ; **JØRGENSEN, J. T.** ; **SHALGUNOV, V.** ; **KJAER, A.** ; **HERTH, M. M**. Pretargeting in Nuclear Imaging and Radionuclide Therapy: Improving Efficacy of Theranostics and Nanomedicines. *Biomaterials*, 2018, vol. 179, 209-245